# EUROPEAN PATENT SPECIFICATION

(11) **EP 1 317 278 B1**
(45) Date of publication and mention of the grant of the patent: **11.11.2009**
(21) Application number: 01973008.4
(22) Date of filing: 11.09.2001
(51) Int. Cl.: A61K 39/00, C07K 16/18, A61K 38/17, A61K 47/48, A61K 51/10, A61K 31/00, G01N 33/50

(54) **MUC1 EXTRACELLULAR DOMAIN AND CANCER TREATMENT COMPOSITIONS AND METHODS DERIVED THEREFROM**
MUC1 EXTRAZELLULÄRE DOMÄNE UND ZUSAMMENSETZUNGEN FÜR KREBSBEHANDLUNG, UND DAVON ABGELEITETE VERFAHREN
DOMAINE EXTRACELLULAIRE DU MUC1 ET COMPOSITIONS ET PROCEDES POUR LE TRAITEMENT DU CANCER DERIVES DE CELUI-CI

(30) Priority: 11.09.2000 US 231841 P
(43) Date of publication of application: 11.06.2003
(73) Proprietor: DANA-FARBER CANCER INSTITUTE, INC., Boston, MA 02115 (US); GENZYME CORPORATION, Cambridge, MA 02142 (US)
(72) Inventor: Kufe, Donald W., Wellesley, MA 02181 (US); Ohno, Tsuneya, Tokyo 105 (JP)
(74) Representative: Dehmel, Albrecht
(86) International application number: PCT/US2001/028548
(87) International publication number: WO 2002/022685

(56) References cited:
- WO-A-00/25827
- WO-A-93/20841
- P. CIBOROWSKI ET AL.: "Screening of anti-Muc1 antibodies for reactivity with native (ascites) and recombinant (baculovirus) Muc1 and for blocking Muc1 specific cytotoxic T-lymphocytes." TUMOR BIOLOGY, vol. 19, no. suppl. 1, 1998, pages 147-151, XP002071242 Basel, Switzerland
- C. TONDINI ET AL.: "Evaluation of monoclonal antibody DF3 conjugated with ricin as a specific immunotoxin for in vitro purging of human bone marrow." CANCER RESEARCH, vol. 50, no. 4, 15 February 1990 (1990-02-15), pages 1170-1175, XP008004616 Baltimore, MD, USA
- S. HIGASHIYAMA ET AL.: "A novel brain-derived member of the epidermal growth factor family that interacts with ErbB3 and ErbB4." JOURNAL OF BIOCHEMISTRY, vol. 122, no. 3, 1997, pages 675-680, XP000775819 Tokyo, JP
- T. NAKASHIMA ET AL.: "Inhibition of angiogenesis by a new isocoumarin, NM-3." THE JOURNAL OF ANTIBIOTICS, vol. 52, no. 4, April 1999 (1999-04), pages 426-428, XP000940445 Tokyo, Japan

### SEQUENCE LISTING

<110> Kufe, Donald W.
   Ohno, Tsuneya
<120> MUC1 EXTRACELLULAR DOMAIN AND CANCER TREATMENT COMPOSITIONS AND METHODS DERIVED THEREFROM
<130> MUC1 APPLICATION
<150> 60/231,841
   <151> 2000-09-11
<160> 18
<170> PatentIn version 3.1
<210> 1
   <211> 45
   <212> PRT
   <213> Homo sapiens
<400> 1
   Thr Ile Asn Val His Asp Val Glu Thr Gln Phe Asn Gln Tyr Lys Thr
   1               5                   10                  15
   Glu Ala Ala Ser Arg Tyr Asn Leu Thr Ile Ser Asp Val Ser Val Ser
               20                  25                  30
   Asp Val Pro Phe Pro Phe Ser Ala Gln Ser Gly Ala Gly
           35                  40                  45
<210> 2
   <211> 426
   <212> PRT
   <213> Homo sapiens
<400> 2
   Met Arg Gln Val Cys Cys Ser Ala Leu Pro Pro Pro Pro Leu Glu Lys
   1 5 10 15
   Gly Arg Cys Ser Ser Tyr Ser Asp Ser Ser Ser Ser Ser Ser Glu Arg
   20 25 30
   Ser Ser Ser Ser Ser Ser Ser Ser Ser Glu Ser Gly Ser Ser Ser Arg
   35 40 45
   Ser Ser Ser Asn Asn Ser Ser Ile Ser Arg Pro Ala Ala Pro Pro Glu
   50 55 60
   Pro Arg Pro Gln Gln Gln Pro Gln Pro Arg Ser Pro Ala Ala Arg Arg
   65 70 75 80
   Ala Ala Ala Arg Ser Arg Ala Ala Ala Ala Gly Gly Met Arg Arg Asp
   85 90 95
   Pro Ala Pro Gly Phe Ser Met Leu Leu Phe Gly Val Ser Leu Ala Cys
   100 105 110
   Tyr Ser Pro Ser Leu Lys Ser Val Gln Asp Gln Ala Tyr Lys Ala Pro
   115 120 125
   Val Val Val Glu Gly Lys Val Gln Gly Leu Val Pro Ala Gly Gly Ser
   130 135 140
   Ser Ser Asn Ser Thr Arg Glu Pro Pro Ala Ser Gly Arg Val Ala Leu
   145 150 155 160
   Val Lys Val Leu Asp Lys Trp Pro Leu Arg Ser Gly Gly Leu Gln Arg
   165 170 175
   Glu Gln Val Ile Ser Val Gly Ser Cys Val Pro Leu Glu Arg Asn Gln
   180 185 190
   Arg Tyr Ile Phe Phe Leu Glu Pro Thr Glu Gln Pro Leu Val Phe Lys
   195 200 205
   Thr Ala Phe Ala Pro Leu Asp Thr Asn Gly Lys Asn Leu Lys Lys Glu
   210 215 220
   Val Gly Lys Ile Leu Cys Thr Asp Cys Ala Thr Arg Pro Lys Leu Lys
   225 230 235 240
   Lys Met Lys Ser Gln Thr Gly Gln Val Gly Glu Lys Gln Ser Leu Lys
   245 250 255
   Cys Glu Ala Ala Ala Gly Asn Pro Gln Pro Ser Tyr Arg Trp Phe Lys
   260 265 270
   Asp Gly Lys Glu Leu Asn Arg Ser Arg Asp Ile Arg Ile Lys Tyr Gly
   275 280 285
   Asn Gly Arg Lys Asn Ser Arg Leu Gln Phe Asn Lys Val Lys Val Glu
   290 295 300
   Asp Ala Gly Glu Tyr Val Cys Glu Ala Glu Asn Ile Leu Gly Lys Asp
   305 310 315 320
   Thr Val Arg Gly Arg Leu Tyr Val Asn Ser Val Ser Thr Thr Leu Ser
   325 330 335
   Ser Trp Ser Gly His Ala Arg Lys Cys Asn Glu Thr Ala Lys Ser Tyr
   340 345 350
   Cys Val Asn Gly Gly Val Cys Tyr Tyr Ile Glu Gly Ile Asn Gln Leu
   355 360 365
   Ser Cys Lys Cys Pro Asn Gly Phe Phe Gly Gln Arg Cys Leu Glu Lys
   370 375 380
   Leu Pro Leu Arg Leu Tyr Met Pro Asp Pro Lys Gln Ser Val Leu Trp
   385 390 395 400
   Asp Thr Pro Gly Thr Gly Val Ser Ser Ser Gln Trp Ser Thr Ser Pro
   405 410 415
   Lys Pro Arg Ser Cys Thr Arg Arg Gly Ser
   420 425
<210> 3
   <211> 422
   <212> PRT
   <213> Homo sapiens
<400> 3
   Met Arg Gln Val Cys Cys Ser Ala Leu Pro Pro Pro Pro Leu Glu Lys
   1 5 10 15
   Gly Arg Cys Ser Ser Tyr Ser Asp Ser Ser Ser Ser Ser Ser Glu Arg
   20 25 30
   Ser Ser Ser Ser Ser Ser Ser Ser Ser Glu Ser Gly Ser Ser Ser Arg
   35 40 45
   Ser Ser Ser Asn Asn Ser Ser Ile Ser Arg Pro Ala Ala Pro Pro Glu
   50 55 60
   Pro Arg Pro Gln Gln Gln Pro Gln Pro Arg Ser Pro Ala Ala Arg Arg
   65 70 75 80
   Ala Ala Ala Arg Ser Arg Ala Ala Ala Ala Gly Gly Met Arg Arg Asp
   85 90 95
   Pro Ala Pro Gly Phe Ser Met Leu Leu Phe Gly Val Ser Leu Ala Cys
   100 105 110
   Tyr Ser Pro Ser Leu Lys Ser Val Gln Asp Gln Ala Tyr Lys Ala Pro
   115 120 125
   Val Val Val Glu Gly Lys Val Gln Gly Leu Val Pro Ala Gly Gly Ser
   130 135 140
   Ser Ser Asn Ser Thr Arg Glu Pro Pro Ala Ser Gly Arg Val Ala Leu
   145 150 155 160
   Val Lys Val Leu Asp Lys Trp Pro Leu Arg Ser Gly Gly Leu Gln Arg
   165 170 175
   Glu Gln Val Ile Ser Val Gly Ser Cys Val Pro Leu Glu Arg Asn Gln
   180 185 190
   Arg Tyr Ile Phe Phe Leu Glu Pro Thr Glu Gln Pro Leu Val Phe Lys
   195 200 205
   Thr Ala Phe Ala Pro Leu Asp Thr Asn Gly Lys Asn Leu Lys Lys Glu
   210 215 220
   Val Gly Lys Ile Leu Cys Thr Asp Cys Ala Thr Arg Pro Lys Leu Lys
   225 230 235 240
   Lys Met Lys Ser Gln Thr Gly Gln Val Gly Glu Lys Gln Ser Leu Lys
   245 250 255
   Cys Glu Ala Ala Ala Gly Asn Pro Gln Pro Ser Tyr Arg Trp Phe Lys
   260 265 270
   Asp Gly Lys Glu Leu Asn Arg Ser Arg Asp Ile Arg Ile Lys Tyr Gly
   275 280 285
   Asn Gly Arg Lys Asn Ser Arg Leu Gln Phe Asn Lys Val Lys Val Glu
   290 295 300
   Asp Ala Gly Glu Tyr Val Cys Glu Ala Glu Asn Ile Leu Gly Lys Asp
   305 310 315 320
   Thr Val Arg Gly Arg Leu Tyr Val Asn Ser Val Ser Thr Thr Leu Ser
   325 330 335
   Ser Trp Ser Gly His Ala Arg Lys Cys Asn Glu Thr Ala Lys Ser Tyr
   340 345 350
   Cys Val Asn Gly Gly Val Cys Tyr Tyr Ile Glu Gly Ile Asn Gln Leu
   355 360 365
   Ser Cys Lys Cys Pro Asn Gly Phe Phe Gly Gln Arg Cys Leu Glu Lys
   370 375 380
   Leu Pro Leu Arg Leu Tyr Met Pro Asp Pro Lys Gln Ser Val Leu Trp
   385 390 395 400
   Asp Thr Pro Gly Thr Gly Val Ser Ser Ser Gln Trp Ser Thr Ser Pro
   405 410 415
   Ser Thr Leu Asp Leu Asn
   420
<210> 4
   <211> 17
   <212> PRT
   <213> Artificial Sequence
<220>
   <223> SYNTHESIZED SEQUENCE
<400> 4
   His Asp Val Glu Thr Gln Phe Asn Gln Tyr Lys Thr Glu Ala Ala Ser
   1 5 10 15
   Cys
<210> 5
   <211> 16
   <212> PRT
   <213> Artificial Sequence
<220>
   <223> SYNTHESIZED SEQUENCE
<400> 5
   His Asp Val Glu Thr Gln Phe Asn Gln Tyr Lys Thr Glu Ala Ala Ser
   1 5 10 15
<210> 6
   <211> 17
   <212> PRT
   <213> Artificial Sequence
<220>
   <223> SYNTHESIZED SEQUENCE
<400> 6
   Gln Tyr Lys Thr Glu Ala Ala Ser Arg Tyr Asn Leu Thr Ile Ser Asp
   1 5 10 15
   Cys
<210> 7
   <211> 19
   <212> PRT
   <213> Artificial Sequence
<220>
   <223> SYNTHESIZED SEQUENCE
<400> 7
   Ser Asp Val Ser Val Ser Asp Val Pro Phe Pro Phe Ser Ala Gln Ser
   1 5 10 15
   Gly Ala Cys
<210> 8
   <211> 19
   <212> PRT
   <213> Artificial Sequence
<220>
   <223> SYNTHESIZED SEQUENCE
<400> 8
   Asp Val Glu Thr Gln Phe Asn Gln Tyr Lys Thr Glu Ala Ala Ser Arg
   1 5 10 15
   Tyr Asn Leu
<210> 9
   <211> 20
   <212> PRT
   <213> Artificial Sequence
<220>
   <223> SYNTHESIZED SEQUENCE
<400> 9
   Asn Ile Lys Phe Arg Pro Gly Ser Val Val Val Gln Leu Thr Leu Ala
   1 5 10 15
   Phe Arg Glu Cys
   20
<210> 10
   <211> 1054
   <212> DNA
   <213> Homo sapiens
<400> 10
   ccctgcagaa gccgcgctcc ctactcggtc cgggggcaga gggggcgcga gagagcaagt 60
   gggcgggcgt cccatcctcc gcatcctcct ccaggtcctg gcgcacaggg tgggagcgct 120
   gcgctgcgcc gcgctgcgca tcgcggcccg cttgccgcct gccccctgcc ctagctgggc 180
   cacctccccg ggctgccggt ggagggctaa gaggcgctaa cgttacgctg tttccggttt 240
   tccagcgggc tctgtttccc ctcccaaggc ggcggcggct gagcggcgga gccccccaaa 300
   tggcctggcc agatgcggca ggtttgctgc tcagcgctgc cgccgccgcc actggagaag 360
   ggtcggtgca gcagctacag cgacagcagc agcagcagca gcgagaggag cagcagcagc 420
   agcagcagca gcagcgagag cggcagcagc agcaggagca gcagcaacaa cagcagcatc 480
   tctcgtcccg ctgcgccccc agagccgcgg ccgcagcaac agccgcagcc ccgcagcccc 540
   gcagcccgga gagccgccgc ccgttcgcga gccgcagccg ccggcggcat gaggcgcgac 600
   ccggcccccg gcttctccat gctgctcttc ggtgtgtcgc tcgcctgcta ctcgcccagc 660
   ctcaagtcag tgcaggacca ggcgtacaag gcacccgtgg tggtggaggg caaggtacag 720
   gggctggtcc cagccggcgg ctccagctcc aacagcaccc gagagccgcc cgcctcgggt 780
   cgggtggcgt tggtaaaggt gctggacaag tggccgctcc ggagcggggg gctgcagcgc 840
   gagcaggtga tcagcgtggg ctcctgtgtg ccgctcgaaa ggaaccagcg ctacatcttt 900
   ttcctggagc ccacggaaca gcccttagtc tttaagacgg cctttgcccc cctcgatacc 960
   aacggcaaaa atctcaagaa agaggtgggc aagatcctgt gcactgactg cggtgagtcg 1020
   ccccctccct ttgctggaga aaggggggag gggc 1054
<210> 11
   <211> 419
   <212> DNA
   <213> Homo sapien
<400> 11
   gcactgcccc taccaccacc gtgctcacct acctgccttg taacccacag ccacccggcc 60
   caagttgaag aagatgaaga gccagacggg acaggtgggt gagaagcaat cgctgaagtg 120
   tgaggcagca gccggtaatc cccagccttc ctaccgttgg ttcaaggatg gcaaggagct 180
   caaccgcagc cgagacattc gcatcaaata tggcaacggc aggtaagcaa tatccagcct 240
   gtcctccatc caagcagctg ccctccctcc tctcgagaag gagtggcctg aactgggcag 300
   aggccagggc tgtgccccca gactcaccca tggaaccagt cccagcccct ctccatactc 360
   cccttccttg ccactcccag ccttcttcct gcagctcggg gaccctctgg tcatggtgg 419
<210> 12
   <211> 493
   <212> DNA
   <213> Homo sapien
<400> 12
   gccactagtc ctgccctgtg ttgtcctaag ggcccaaggc tccctgcagc tgcagggact 60
   ccgtgcccca cctggactcc agaaattgct ggggggaggg aatctccttc ccatctaagc 120
   tgcagggcca ctgtggctct gtggagagag gcaaccgctg ggtgactgct ggggagccac 180
   agccggccct ggctcacgcc tctccccctt ttatcccctc cctaaccaga aagaactcac 240
   gactacagtt caacaaggtg aaggtggagg acgctgggga gtatgtctgc gaggccgaga 300
   acatcctggg gaaggacacc gtccggggcc ggctttacgt caacagcggt aggtgggccc 360
   agacagaggg aagggtccta gaggggtttg gcagggcggg ccagtggccc cccagccctt 420
   ggggcctgca tgtgatccat ctcgcactca actcccctcc ctcctgctgc tgccatatca 480
   agccctagat ctc 493
<210> 13
   <211> 350
   <212> DNA
   <213> Homo sapien
<400> 13
   ggtcatcttc cagttttgac gtggggcatg aaggagatga ttcctggggc ctagggatag 60
   tctcagtgcg tcactggcac atgtctctca taccctcagt gagcaccacc ctgtcatcct 120
   ggtcggggca cgcccggaag tgcaacgaga cagccaagtc ctattgcgtc aatggaggcg 180
   tctgctacta catcgagggc atcaaccagc tctcctgcaa gtaagtgacc agtaggggtg 240
   ggcatgggag caagaacagg gtaggagatg ctgggtcaga agtggagggc tctaggaaaa 300
   gagggttcca agccactgac aagaggtccc caaggggtgt agacaggaag 350
<210> 14
   <211> 326
   <212> DNA
   <213> Homo sapien
<400> 14
   aagtgcctga cttggttttt ctcattcatc actgtgcact ttgatttcta accttagcct 60
   cgcggatgat gccagtttgt tctgtgattt ttcccctttt ccttctccag atgtccaaat 120
   ggattcttcg gacagagatg tttggagaaa ctgcctttgc gattgtacat gccagatcct 180
   aagcaaagta tgtttgaaga tacaaacaca agtccctgct ccttagaaag cttctgggtg 240
   cagtcccccc aatgtagggc atagggccag gggtgttggt tgttttgatc tttggctgtt 300
   ttgttttgtt tctcttttct tttttg 326
<210> 15
   <211> 310
   <212> DNA
   <213> Homo sapien
<400> 15
   aatacaaatg gtaacggtgg caaggaaccc agctggcatt ggcaggagcc cgtgccgggg 60
   tgttgcaatg cctgtgtctt gctatcctgg ctctctcttt ctggttttct ttcttttggt 120
   aggtgtcctg tgggatacac cggggacagg tgtcagcagt tcgcaatggt caacttctcc 180
   agtatgtctc tttcttctat ttcttctctt ccctggtgac tgacagtctc cccctgggga 240
   gggatggggc cttgcttagc aagactaggg ggagatgtag agggtgtcct gcgctcctta 300
   gaggagacgg 310
<210> 16
   <211> 259
   <212> DNA
   <213> Homo sapien
<400> 16
   ggatggaaag gagctgggca ggacctgagc tggggagagc ctatggcttt gggggcaaag 60
   gggtctctgc accactatcc ctatggggtt tgcctcctgc cccacccctc acatctaccc 120
   tacctctgca gaagccgagg agctgtacca gaagagggtc ctgaccatca cgggcatctg 180
   cgtggctctg ctggtcgtgg gcatcgtctg tgtggtggcc tactgcaaga ccaagtgagt 240
   gtcactcact caagctggg 259
<210> 17
   <211> 258
   <212> DNA
   <213> Homo sapien
<400> 17
   ttgtgaccct cgctcttctg cactgccaga gcaccttgga tttgaattaa agggtatgaa 60
   acctctgtgt gtcactcagc accatctgcg tggcccctct ccacagcctc cgatgccatg 120
   tgtgctgaat gtttggtttc ctttccaaaa aatggaaaag ggatgattcc aaaaatctct 180
   cctggcccca tcgagatgtc ctatcataaa ctacgttgat ttagcaggag acgaaagaag 240
   ctcatgaaca gaagggca 258
<210> 18
   <211> 8181
   <212> DNA
   <213> Homo sapien
<220>
   <221> misc_feature
   <222> (6890)..(7960)
   <223> definition of "n" in this sequence is unavailable at this time
<220>
   <221> misc_feature
   <222> (6890)..(7960)
   <223> unknown nucleotide
<400> 18
   gaattcagaa ttttagaccc tttggccttg gggtccatcc tggagaccct gaggtctaag 60
   ctacagcccc tcagccaacc acagaccctt ctctggctcc caaaaggagt tcagtcccag     120
   agggtggtca cccacccttc agggatgaga agttttcaag gggtattact caggcactaa     180
   ccccaggaaa gatgacagca cattgccata aagttttggt tgttttctaa gccagtgcaa     240
   ctgcttattt tagggatttt ccgggatagg gtggggaagt ggaaggaatc ggcgagtaga     300
   agagaaagcc tgggagggtg gaagttaggg atctagggga agtttggctg atttggggat     360
   gcgggtgggg gaggtgctgg atggagttaa gtgaaggata gggtgcctga gggaggatgc     420
   ccgaagtcct cccagaccca cttactcacg gtggcagcgg cgacactcca gtctatcaaa     480
   gatccgccgg gatggagagc caggaggcgg gggctgcccc tgaggtagcg gggaggccgg     540
   ggggccgggg ggcggacggg acgagtgcaa tattggcggg ggaaaaaaca acactgcacc     600
   gcgtcccgtc cctcccgccc gcccgggccc ggatcccgct ccccaccgcc tgaagccggc     660
   ccgacccgga acccgggccg ctggggagtt gggttcacct tggaggccag agagacttgg     720
   cgcccggaag caaagggaat ggcaaggggg aggggggagg gagaacggga gtttgcggag     780
   tccagaaggc cgctttccga cgcccgggcg ttgcgcgcgc ttgctcttta agtactcaga     840
   ctgcgcggcg cgagccgtcc gcatggtgac gcgtgtccca gcaaccgaac tgaatggctg     900
   ttgcttggca atgccgggag ttgaggtttg gggccgccca cctagctact cgtgttttct     960
   ccggcctgcg agttgggggg ctcccgcctc cccggcccgc tcctgggcgc gctgacgtca    1020
   gatgtcccca ccccgcccag cgcctgcccc aagggtctcg ccgcacacaa agctcggcct    1080
   cgggcgccgg cgcgcgggcg agagcggtgg tctctcgcct gctgatctga tgcgctccaa    1140
   tcccgtgcct cgccgaagtg tttttaaagt gttctttcca acctgtgtct ttggggctga    1200
   gaactgtttt ctgaatacag gcggaactgc ttccgtcggc ctagaggcac gctgcgactg 1260
   cgggacccaa gttccacgtg ctgccgcggc ctgggatagc ttcctcccct cgtgcactgc 1320
   tgccgcacac acctcttggc tgtcgcgcat tacgcacctc acgtgtgctt ttgccccccg 1380
   ctacgtgcct acctgtcccc aataccactc tgctccccaa aggatagttc tgtgtccgta 1440
   aatcccattc tgtcacccca cctactctct gcccccccct tttttgtttt gagacggagc 1500
   tttgctctgt cgcccaggct ggagtgcaat ggcgcgatct cggctcactg caacctccgc 1560
   ctcccgggtt caagcgattc tcctgcctca gcctcctgag tagctggggt tacagcgccc 1620
   gccaccacgc tcggctaatt tttgtagttt ttagtagaga cgaggtttca ccatcttggc 1680
   caggctggtc ttgaacccct gaccttgtga tccactcgcc tcggccttcc aaagtgttgg 1740
   gattacgggc gtgacgaccg tgccacgcat ctgcctctta agtacataac ggcccacaca 1800
   gaacgtgtcc aactcccccg cccacgttcc aacgtcctct cccacatacc tcggtgcccc 1860
   ttccacatac ctcaggaccc cacccgctta gctccatttc ctccagacgc caccaccacg 1920
   cgtcccggag tgccccctcc taaagctccc agccgtccac catgctgtgc gttcctccct 1980
   ccctggccac ggcagtgacc cttctctccc gggccctgct tccctctcgc gggctctgct 2040
   gcctcactta ggcagcgctg cccttactcc tctccgcccg gtccgagcgg cccctcagct 2100
   tcggcgccca gccccgcaag gctcccggtg accactagag ggcgggagga gctcctggcc 2160
   agtggtggag agtggcaagg aaggacccta gggttcatcg gagcccaggt ttactccctt 2220
   aagtggaaat ttcttccccc actcctcctt ggctttctcc aaggagggaa cccaggctgc 2280
   tggaaagtcc ggctgggggg gggactgtgg gttcagggga gaacggggtg tggaacggga 2340
   cagggagcgg ttagaagggt ggggctattc cgggaagtgg tggggggagg gagcccaaaa 2400
   ctagcaccta gtccactcat tatccagccc tcttatttct cggccgctct gcttcagtgg 2460
   acccggggag ggcggggaag tggagtggga gacctagggg tgggcttccc gaccttgctg 2520
   tacaggacct cgacctagct ggctttgttc cccatcccca cgttagttgt tgccctgagg 2580
   ctaaaactag agcccagggg ccccaagttc cagactgccc ctcccccctc ccccggagcc 2640
   agggagtggt tggtgaaagg gggaggccag ctggagaaca aacgggtagt cagggggttg 2700
   agcgattaga gcccttgtac cctacccagg aatggttggg gaggaggagg aagaggtagg 2760
   aggtagggga gggggcgggg ttttgtcacc tgtcacctgc tcgctgtgcc tagggcgggc 2820
   gggcggggag tggggggacc ggtataaagc ggtaggcgcc tgtgcccgct ccacctctca 2880
   agcagccagc gcctgcctga atctgttctg ccccctcccc acccatttca ccaccaccat 2940
   gacaccgggc acccagtctc ctttcttcct gctgctgctc ctcacagtgc ttacaggtga 3000
   ggggcacgag gtggggagtg ggctgccctg cttaggtggt cttcgtggtc tttctgtggg 3060
   ttttgctccc tggcagatgg caccatgaag ttaaggtaag aattgcagac agaggctgcc 3120
   ctgtctgtgc cagaaggagg gagaggctaa ggacaggctg agaagagttg cccccaaccc 3180
   tgagagtggg taccaggggc aagcaaatgt cctgtagaga agtctagggg gaagagagta 3240
   gggagaggga aggcttaaga ggggaagaaa tgcaggggcc atgagccaag gcctatgggc 3300
   agagagaagg aggctgctgc agggaaggag gcttccaacc caggggttac tgaggctgcc 3360
   cactccccag tcctcctggt attatttctc tggtggccag agcttatatt ttcttcttgc 3420
   tcttattttt ccttcataaa gacccaaccc tatgacttta acttcttaca gctaccacag 3480
   cccctaaacc cgcaacagtt gttacaggtt ctggtcatgc aagctctacc ccaggtggag 3540
   aaaaggagac ttcggctacc cagagaagtt cagtgcccag ctctactgag aagaatgctg 3600
   tgagtatgac cagcagcgta ctctccagcc acagccccgg ttcaggctcc tccaccactc 3660
   agggacagga tgtcactctg gccccggcca cggaaccagc ttcaggttca gctgccacct 3720
   ggggacagga tgtcacctcg gtcccagtca ccaggccagc cctgggctcc accaccccgc 3780
   cagcccacga tgtcacctca gccccggaca acaagccagc cccgggctcc accgcccccc 3840
   cagcccacgg tgtcacctcg gccccggaca ccaggccggc cccgggctcc accgcccccc 3900
   cagcccatgg tgtcacctcg gccccggaca acaggcccgc cttgggctcc accgcccctc 3960
   cagtccacaa tgtcacctcg gcctcaggct ctgcatcagg ctcagcttct actctggtgc 4020
   acaacggcac ctctgccagg gctaccacaa ccccagccag caagagcact ccattctcaa 4080
   ttcccagcca ccactctgat actcctacca cccttgccag ccatagcacc aagactgatg 4140
   ccagtagcac tcaccatagc acggtacctc ctctcacctc ctccaatcac agcacttctc 4200
   cccagttgtc tactggggtc tctttctttt tcctgtcttt tcacatttca aacctccagt 4260
   ttaattcctc tctggaagat cccagcaccg actactacca agagctgcag agagacattt 4320
   ctgaaatggt gagtatcggc ctttccttcc ccatgctccc ctgaagcagc catcagaact 4380
   gtccacaccc tttgcatcaa gcccgagtcc tttccctctc accccagttt ttgcagattt 4440
   ataaacaagg gggttttctg ggcctctcca atattaagtt caggtacagt tctgggtgtg 4500
   gacccagtgt ggtggttgga gggttgggtg gtggtcatga ccgtaggagg gactggtgca 4560
   cttaaggttg ggggaagagt gctgagccag agctgggacc cgtggctgaa gtgcccattt 4620
   ccctgtgacc aggccaggat ctgtggtggt acaattgact ctggccttcc gagaaggtac 4680
   catcaatgtc cacgacgtgg agacacagtt caatcagtat aaaacggaag cagcctctcg 4740
   atataacctg acgatctcag acgtcagcgg tgaggctact tccctggctg cagccagcac 4800
   catgccgggg cccctctcct tccagtgtct gggtccccgc tctttcctta gtgctggcag 4860
   cgggaggggc gcctcctctg ggagactgcc ctgaccactg cttttccttt tagtgagtga 4920
   tgtgccattt cctttctctg cccagtctgg ggctggggtg ccaggctggg gcatcgcgct 4980
   gctggtgctg gtctgtgttc tggttgcgct ggccattgtc tatctcattg ccttggtgag 5040
   tgcagtccct ggccctgatc agagcccccc ggtagaaggc actccatggc ctgccataac 5100
   ctcctatctc cccaggctgt ctgtcagtgc cgccgaaaga actacgggca gctggacatc 5160
   tttccagccc gggataccta ccatcctatg agcgagtacc ccacctacca cacccatggg 5220
   cgctatgtgc cccctagcag taccgatcgt agcccctatg agaaggtgag attgggcccc 5280
   acaggccagg ggaagcagag ggtttggctg ggcaaggatt ctgaaggggg tacttggaaa 5340
   acccaaagag cttggaagag gtgagaagtg gcgtgaagtg agcaggggag ggcctggcaa 5400
   ggatgagggg cagaggtcag aggagttttg ggggacaggc ctgggaggag actatggaag 5460
   aaaggggccc tcaagaggga gtggccccac tgccagaatt cctaaaagat cattggccgt 5520
   ccacattcat gctggctggc gctggctgaa ctggtgccac cgtggcagtt ttgttttgtt 5580
   ttgctttttt gcacccagag gcaaaatggg tggagcacta tgcccagggg agcccttccc 5640
   gaggagtccc aggggtgagc ctctgtgccc ctaatcatct cctaggaatg gagggtagac 5700
   cgagaaaggc tggcataggg ggaggtttcc caggtagaag caagaagaag tgtcagcaga 5760
   ccaggtgagc gtgggtgcca gtggggttct tgggagcttc aaggaagcaa ggaacgctcc 5820
   ctccttcctc tcctggtctt tctctatggg acctagtaaa taattactgc agccacctga 5880
   ggctggaaaa ccactccagg tgggggagga gagagtttag ttttcttgct cctattttcc 5940
   tcctcctgga gacctccctc tctcggcttt acaaagacac agatacaccc cgccccccaa 6000
   aacacacaca cacacacaca cacacacacc tccttaggct ggaacagcag agaatggagg 6060
   gacaaggggg ctgattagag ccaagaagag ggagtgaagg agagcagagg gaggagggca 6120
   gccctgttta cagtcacctg gctggtgggg tggcaggtgc tctctctgaa ttaacccttt 6180
   gagagctggc caggactctg gactgattac cccagcctgg ggtggcatcc aggggctcta 6240
   ggaggtacct tttgctcctc accctggatc tcttttcctt ccacccaggt ttctgcaggt 6300
   aatggtggca gcagcctctc ttacacaaac ccagcagtgg cagccacttc tgccaacttg 6360
   taggggcacg tcgcccgctg agctgagtgg ccagccagtg ccattccact ccactcaggt 6420
   tcttcagggc cagagcccct gcaccctgtt tgggctggtg agctgggagt tcaggtgggc 6480
   tgctcacacg tccttcagag gccccaccaa tttctcggac acttctcagt gtgtggaagc 6540
   tcatgtgggc ccctgaggct catgcctggg aagtgttgtg gtgggggctc ccaggaggac 6600
   tggcccagag agccctgaga tagcggggat cctgaactgg actgaataaa acgtggtctc 6660
   ccactggcgc caacttctga tctttcatct gtgacccgtg ggcagcaggg cgtcagaatg 6720
   tgtgtgaggg ggctggggga ggagacaggg aggccaggag gcagtaagga gcgagtttgt 6780
   ttgagaagca ggagatgtga ggaggaggtg acattgggga gtaggggtgg cctgaggagc 6840
   cacctctggc taaccctggc agcacaagag gaaggaggaa acgaaaccca ggcnggcttt 6900
   ggagggctag cgtgactggg ctccgtgact gagctctgtg tgccagtggc tctcccctct 6960
   cctcgcctgg cccacgccct ccttgcccct ggcatggtgc cccccaggtg gctctattct 7020
   tagctgtccg ggtgtgaagt aaatccttgg gcagtgataa cagcccagag tcaacagggt 7080
   tgagataagc agaggctggg tcagatccgg gcgctggcac caggcccagc cccctccctg 7140
   accccggctn ccccaccagc ctgctgcccc tggggtggnc tccacaacac cctgggaatg 7200
   gggaagtggt tctggttccc tgaccccttt ggcccaggca cgttgcctgt ccctcgaccg 7260
   cattccccca gggcctgtgc tgcaggcctg gaagccctga ttggggcctg ccaccagcag 7320
   ccagagagct atgttccctg gcagctgtga tgcgctcagg ccgggccagg acacgtgtgg 7380
   caggaggctt agagcacctg cctggggcct tcctctctca ggcaccagat ccattggttg 7440
   ctcctgccta gaaccacagc ctagcacccc tgctccctcc cgcctaccac acccagcaca 7500
   gaaactcaca ggaatgattg cgctcaggga aggcagagat gtgcctggca tcacagttta 7560
   ttgtttataa accatgacaa taacagctgt tgctcagcac aggcctagca gagcccactg 7620
   cagggggacg gcagcgggca ccagaggcct tgcctggccc aacccaatgg gaacacccag 7680
   actcagctgg gtccccaagg gagacttggc acattggcat gggtgtggga caggtaaagc 7740
   atgcaagagg gagaagaggg acataagggg catgcggctg cggggtgttg ggacccaaat 7800
   aaataaagca ggatgacagg gtccccttcc cctcaccagg aatgcctgac agcgtccagc 7860
   cccaaagcct gcctgtccca aggctgtagt tcagcatcaa cagggcaggg agcttggcag 7920
   ggcaagggca gagctggaga tcatgcccag tnttccaggt gccctccctc ccaatcagcc 7980
   tggggggcac aggacaggga tggagaaggg gctctctcca tggcttgggt aacatgccaa 8040
   aggcaggtca tagggcagac tcagtggggg tgggggcctg gctaacaagc aatggagaga   8100
   acgggggcca tccagagagg ttggcagaag agagcccctg ggtcaagaga aaactttggg   8160
   gaagacaaga cacgggagaa g                                             8181

## Description

### FIELD OF THE INVENTION

The present invention relates generally to the field of cancer therapy and more specifically to the use of modulators or agents that interact with MUC 1 as a point on intervention in cancer therapy.

### BACKGROUND OF THE INVENTION

The human MUC1 mucin glycoprotein is expressed on the apical borders of secretory epithelial cells on the luminal surface of most glandular epithelia (Kufe *et al*., 1984). In carcinomas, MUC1 is highly overexpressed throughout the entire cell membrane and cytoplasm (Kufe *et al*., 1984; Perey *et al*., 1992). As such, the aberrant pattern of MUC expression in carcinoma cells may confer a function for MUC1 normally found at the apical membrane to the entire cell membrane. The hallmark of MUC1 mucin is an ectodomain comprising a glycosylated 20 amino acid extracellular sequence that is tandemly repeated 25-100 times in each molecule (Strouss & Decker, 1992). The mucin glycosylation level appears to be lower in cancer cells than normal cells of ductal epithelial tissue (Kufe, U.S. Pat. No. 5,506,343). This hypoglycosylation results in the exposure of tumor-specific epitopes that are hidden in the fully glycosylated mucin.

Over ninety percent of breast cancers show an increased expression of MUC I (also known as Mucin, Epithelial Membrane Antigen, Polymorphic Epithelial Mucin, Human Milk Fat Globule Membrane antigen, Episialin, DF-3, etc., *see* Barry & Sharkey, 1985). Several clinical studies have suggested that mucinous tumor antigens expressed on the cell surface of tumor cells associate with poor prognosis of a variety of cancer types (Itzkowitz *et al*., 1990).

MUC1 is expressed as both a transmembrane form and a secreted form (Finn *et al.,* 1995). The repeating sialyl epitopes of MUC1 (the 'ectodomain') are shed into the serum (Reddish *et. al*., 1996). The N-terminal ectodomain (the extracellular domain that is cleaved) of MUC1 consists of a variable number of the 20-amino acid tandem repeats that are subject to O-glycosylation. This mucin extends far above the cell surface and past the glycocalyx making it easily available for interactions with other cells. The C-terminal region of MUC1 includes a 37 amino acid transmembrane domain and a 72 amino acid cytoplasmic tail that contains sites for tyrosine phosphorylation. A approximately 45-amino acid extracellular domain remains following cleavage of the ectodomain. It is not known what enzyme is responsible for the cleavage of the ectodomain at this time. The extracellular domain or "MUC1/ECD," remaining, after cleavage of the ectodomain, typically includes the amino acid sequence:

TINVHDVETQFNQYKTEAASRYNLTISDVSVSDVPFPFSAQSGAG.

The cytoplasmic domain of MUC1 ("MUC1/CD") encompasses multiple sub-domains that are important in intracellular signaling in cancer cells. β-Catenin binds directly to MUC1/CD at a SAGNGGSSL motif (Yamamoto *et al.,* 1997). β-Catenin, a component of the adherens junctions of mammalian epithelium, binds to cadherins at the intracellular surface of the plasma membrane and performs a signaling role in the cytoplasm as the penultimate downstream mediator of the wnt signaling pathway (Takeichi, 1990; Novak & Dedhar, 1999). The ultimate mediator of the wnt pathway is a nuclear complex of β-catenin and lymphoid enhancer factor/T cell factor (Lef/Tcf) which stimulates the transcription of a variety of target genes (*see e.g*., Molenaar *et al*., 1996; Brunner *et al*., 1997). Defects in the β-catenin-Lef/Tcf pathway are involved in the development of several types of cancers (Novak & Dedhar, 1999).

Glycogen synthase kinase 3β (GSK3β) also binds directly to MUC1/CD and phosphorylates serine in a DRSPY site adjacent to the β-catenin binding motif, thereby decreasing the association between MUC1 and β-catenin (Li *et al*., 1998). In addition, the c-Src tyrosine kinase also binds to and phosphorylates a MLTC1/CD SPYEKV motif, resulting in an increased interaction between MUC1/CD and β-catenin and a decreased interaction between MLJC1/CD and GSK3β (Li *et al.,* 2001).

MUC1 associates also constitutively with the epidermal growth factor receptor (EGF-R, HER1) at the cell membrane and activated EGF-R induces phosphorylation of the MCTC1/CD SPYKEV motif (Li *et al.,* 2001(a)). EGF-R mediated phosphorylation of MUC1/CD appears to increase the interaction of MUC1 with c-Src and β-catenin and downregulate the interaction between MUC1 and GSK3β. These results support a model wherein MUC integrates the signaling among c-Src, β-catenin and GSK3β pathways and dysregulation of this integrated signaling by aberrant overexpression of MLTC1 in cancer cells could promote the transformed phenotype (Li *et al.,* 2001(a)).

The Armadillo protein p120^{ctn} also binds directly to MLJC1/CD resulting in the nuclear localization of p120 (Li & Kufe, 2001). P120 has been implicated in cell transformation and altered patterns of p120 expression have been observed in carcinomas (*see e.g.,* Jawhari *et al.,* 1999; Shimazui *et al.,* 1996). P120 is a v-Src tyrosine kinase substrate, binds to E-cadherin, and is implicated as a transcriptional coactivator (Reynolds *et al*., 1989; Reynolds *et al.,* 1994; Daniels & Reynolds, 1999). The observations that p120 localizes to both cell junctions and the nucleus have supported a role for p120, like β-catenin, in the regulation of both cell adhesion and gene transcription. Decreased cell adhesion resulting from association of MUC1 and p120 may be involved in increased metastatic potential of MUC1-expressing tumor cells.

Thus the available evidence indicates that MUC1/CD functions to transfer signals from the extracellular domain to the nucleus, and utilizes signaling mechanisms that have been implicated in adhesion receptor and growth factor signaling and cellular transformation. It is desirable to identify compositions and methods related to modulation of the MUC1-mediated signaling and its putative role in cellular transformation.

### SUMMARY OF THE INVENTION

The present invention encompasses methods of use and pharmaceutical compositions relating to the discovery that the extracellular domain of MUC I provides binding domains for endogenous ligands and that such binding is related to an oncogenic function of MUC 1 and the proliferation of cancer cells.

Broadly the invention relates to cancer treatment compositions and methods employing agents that comprise or include antagonists of MUC1/ECD modulated cell proliferation that bind to MUC1/ECD or that bind to MUC1/ECD ligands that activate the oncogenic function of MUC 1.

Thus, one aspect of the present invention provides for the manufacture of a medicament for inhibiting the proliferation of cancer cells, the medicament comprising an effective amount of a MUC1/ECD antagonist MUC1/ECD antagonists are MUC1/ECD binding inhibitors. A "MUC1/ECD binding inhibitor" means a compound that inhibits the binding of MUC 1 wild-type ligands, which may suitably include neuregulin 2 isoform 5 (SEQ ID NO: 2), neuregulin 2 isoform 6 (SEQ ID NO: 3), and appropriate fragments thereof, to MUC1/ECD or a compound that inhibits the binding of an antibody that binds to an epitope within SEQ ID NO. 4 to MUC1/ECD. Appropriate fragments of neuregulin 2 isoform 5 (SEQ ID NO: 2) and neuregulin 2 isoform 6 (SEQ ID NO: 3) are those that bind to MUC1/ECD. MUC1/ECD binding inhibitors include antibodies, polypeptides and small molecules that inhibit such binding. A "MUC1/ECD-P1 binding inhibitor" means a MUC1/ECD binding inhibitor identified by inhibition of the binding to MUC1/ECD of an antibody the binds to an epitope within SEQ ID NO. 4.

In one embodiment of the invention, the MUC1/ECD inhibitor is the polypeptide of SEQ ID NO: 1. In another embodiment, the MUC1/CD binding inhibitor is the polypeptide of SEQ ID NO: 4 or, SEQ ID NO: 5.

In another embodiment of the present invention, the MUC1/ECD inhibitor is an antibody that binds to one or more epitopes in the MUC1/ECD sequence SEQ ID NO: 1. The antibody may be a polyclonal or a monoclonal antibody. Monoclonal antibodies may be humanized or human monoclonal antibodies. It may also be a bispecific antibody or a fragment which comprises an antigen binding region. In some embodiments, the antibody is conjugated to a chemotherapeutic agent, radioisotope, toxin, or an effector that induces a cytolytic or cytotoxic immune response. Such conjugates may comprise a cytokine, an antimetabolite, an anthracycline, a vinca alkaloid, an antibiotic, an alkylating agent, a naturally derived toxin, or an Fc region of a IgG1 immunoglobulin.

In another embodiment, the medicament can be formulated for the administration of a chemotherapeutic agent or radiation in combination with a MUC1/ECD antagonist. Chemotherapeutic agents typically include alkylating agents, topoisomerase inhibitors, antimetabolites, tubulin interactive agents, anti-hormonal agents, ornithine decarboxylase inhibitors and tyrosine kinase inhibitors.

The cancer cells are selected from the group consisting of skin cancer cells, prostate cancer cells, lung cancer cells, brain cancer cells, breast cancer cells, ovarian cancer cells, cervical cancer cells, liver cancer cells, pancreatic cancer cells, colon cancer cells, stomach cancer cells and leukemia cells. The tumor to be treated with the medicament can be a tumor of the skin, prostate, lung, brain, breast, ovary, cervix, liver, pancreas, colon, stomach or hematopoetic system.

Other aspects or the invention relate to pharmaceutical compositions comprising MUC1/ECD antagonists and a pharmaceutically acceptable carrier. Wherein the antagonist is a MUC1/ECD binding inhibitor that may be the polypeptide of SEQ ID NO: 1 , and a pharmaceutically acceptable carrier. In some embodiments, the MUC1/ECD inhibitor may be the polypeptide of SEQ ID NO: 4 or; SEQ ID NO: 5. In other embodiments, the pharmaceutical composition comprises an antibody that is a MUC1/ECD binding inhibitor and binds to an epitope within sequences of the peptides of SEQ ID NO: 1, and a pharmaceutically acceptable carrier.

The present invention also encompasses methods for screening MUC1/ECD binding inhibitor activity. One embodiment comprises a method of identifying a compound that inhibits the binding of ligands to MUC1/ECD, the method comprising: (a) providing a polypeptide comprising SEQ ID. NO: I or SEQ ID NO: 5; (b) contacting said polypeptide with a test compound and a ligand to the extracellular domain of MUC1 selected from the group consisting of antibodies to MUC1/ECD that stimulate MUC 1 mediated cancer cell proliferation and wild type ligands that bind to MUC1/ECD and stimulate cancer cell proliferation; and (c) determining whether the binding of said antibody to MUC1/ECD or wild type ligand is decreased relative to an appropriate control. Appropriate controls include, but are not limited to, assays wherein test compounds are excluded. In one embodiment the MUC1/ECD antibody that stimulates MUC 1 mediated cancer cell proliferation is an antibody that binds to an epitope within SEQ ID NO. 4. In other embodiments, the wild type ligands may suitable include neuregulin 2 isoform 5 (SEQ ID NO: 2) and appropriate fragments thereof and neuregulin 2 isoform 6 (SEQ ID NO: 3) and appropriate fragments thereof, wherein appropriate fragments are those that bind to the SEQ ID NO: 1 and have suitable growth stimulatory activity.

Another embodiment is a method of identifying a compound that inhibits the proliferation of MUC1-expressing cancer cells, the method comprising: (a) contacting a population of MUC1-expressing cancer cells obtained previously with a test compound and a ligand to the extracellular domain of MUC1 selected from the group consisting of antibodies to MUC1/ECD that stimulate MUC1 mediated cancer cell proliferation and wild type ligands that bind to MUC1/ECD and stimulate cancer cell proliferation; and (b) determining whether the proliferation of the population of MUC1-expressing cancer cells is decreased by comparison to an appropriate control. Appropriate controls include, but are not limited to, proliferation assays wherein test compounds are excluded. In one embodiment the MUC1/ECD antibody that stimulates MUC1 mediated cancer cell proliferation is an antibody that binds to an epitope within SEQ ID NO. 4. In other embodiments, the wild type ligands may suitable include neuregulin 2 isoform 5 (SEQ ID NO: 2) and appropriate fragments thereof and neuregulin 2 isoform 6 (SEQ ID NO: 3) and appropriate fragments thereof, wherein appropriate fragments are those that bind to the SEQ ID NO: 1 and have suitable growth stimulatory activity.

The present invention also provides methods for identifying compounds that downregulate MUC1/ECD expression. The method comprises: (a) contacting a population of MUC1-expressing cancer cells obtained previously with a test compound; (b) utilizing an anti-MUC1/ECD antibody to identify polypeptides comprising MUC1/ECD in the MUC1-expressing cancer cells; and (c) determining whether the expression of polypeptides comprising MUC1/ECD is decreased in comparison to controls wherein the test compound was excluded.

The present invention also encompasses pharmaceutical compositions comprising compounds identified by the foregoing methods and a pharmaceutically acceptable carrier.

### BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.
**FIG. 1****:** Effect of anti-MUC1-P1 antibody on proliferation of ZR-75-1 breast carcinoma cells.
**FIG. 2****:** Effect of anti-MUC1-P1 antibody on proliferation of SW480 cells stably expressing an empty vector (SW480/V) or MUC1 (SW480/MUC1).
**FIG. 3****:** Effect of ZR-75-1 conditioned medium on proliferation of SW480 cells stably expressing an empty vector (SW480/V) or MUC1 (SW480/MLTC1).
**FIG. 4****:** Effect of MUC1 on H₂O₂ and taxol-induced apoptosis in HeLa cells stably expressing an empty vector (HeLa/V) or MUC1 (HeLa/Muc1). The results are expressed as the percentage apoptosis (mean±SE) of three separate experiments.

### DETAILED DESCRIPTION OF THE INVENTION

### I. Polypeptides

The polypeptides useful in the present invention can be created by synthetic techniques or recombinant techniques which employ genomic or cDNA cloning methods. Polypeptides can be routinely synthesized using solid phase or solution phase peptide synthesis. Methods of preparing relatively short polypeptides peptides, such as P0 (SEQ ID NO: 9), PI (SEQ ID NO: 4), P2 (SEQ ID NO: 6) and P3 (SEQ ID NO: 7), by chemical synthesis are well known in the art. Such polypeptides could, for example be produced by solid-phase peptide synthesis techniques using commercially available equipment and reagents such as those available from Milligen (Bedford, Mass.) or Applied Biosystems-Perkin Elmer (Foster City, CA). Alternatively, segments of such polypeptides could be prepared by solid-phase synthesis and linked together using segment condensation methods such as those described by Dawson *et al*.,(1994). During chemical synthesis of such polypeptides, substitution of any amino acid is achieved simply by replacement of the residue that is to be substituted with a different amino acid monomer.

Wild-type MUC1/ECD ligand polypeptides can be identified as exemplified in Example 3 herein. Recombinant MUC1/ECD ligands can then be prepared by methods known in the art.

Also encompassed by the present invention are chemical derivatives of polypeptides. "Chemical derivative" refers to a subject polypeptide having one or more residues chemically derivatized by reaction of a functional side group. Such derivatized residues include, for example, those molecules in which free amino groups have been derivatized to form amine hydrochlorides, *p*-toluene sulfonyl groups, carbobenzoxy groups, *t*-butyloxycarbonyl groups, chloroacetyl groups or formyl groups. Free carboyl groups may be derivatized to form salts, methyl and ethyl esters or other types of esters or hyrazides. Free hydroxyl groups may be derivatized to form O-acyl or O-alkyl derivatives. The imadazole group of histidine may be derivatized to form N-imbenzylhistidine.

The term "polypeptide" as used herein indicates a molecular chain of amino acids and does not refer to a specific length of the product.

### II. Antibodies

The term "antibody" is used in the broadest sense and specifically covers monoclonal antibodies (including full length monoclonal antibodies), polyclonal antibodies, multispecific antibodies (e.g., bispecific antibodies), and antibody fragments, so long as they exhibit the desired biological activity.

Methods for generating polyclonal antibodies are well known in the art. Briefly, a polyclonal antibody is prepared by immunizing an animal with an antigenic composition and collecting antisera from that immunized animal. A wide range of animal species can be used for the production of antisera including rabbit, mouse, rat, hamster, guinea pig and goat.

As is well known in the art, a given composition may vary in its immunogenicity. It is often necessary therefore to boost the host immune system, as may be achieved by coupling a polypeptide immunogen to a carrier. Exemplary and preferred carriers are keyhole limpet hemocyanin (KLH) and bovine serum albumin (BSA). Other albumins such as ovalbumin, mouse serum albumin or rabbit serum albumin can also be used as carriers. Means for conjugating a polypeptide to a carrier protein are well known in the art and include glutaraldehyde, m-maleimidobenzoyl-N-hydroxysuccinimide ester, carbodiimide and bis-biazotized benzidine. As is also well known in the art, the immunogenicity of a particular immunogen composition can be enhanced by the use of non-specific stimulators of the immune response, known as adjuvants. Exemplary and preferred adjuvants include complete Freund's adjuvant (a non-specific stimulator of the immune response containing killed Mycobacterium tuberculosis), incomplete Freund's adjuvants and aluminum hydroxide adjuvant.

The serum for an immunized animal may be used as is for various applications or the desired antibody fraction may be purified by well-known methods, such as affinity chromatography using another antibody or a peptide bound to a solid matrix.

Monoclonal antibodies (MAbs) may be readily prepared through use of well-known techniques, such as those exemplified in U.S. Patent 4,196,265.

Typically, this technique involves immunizing a suitable animal with a selected immunogen composition, e.g., a purified or partially purified expressed polypeptide. The immunizing composition is administered in a manner that effectively stimulates antibody producing cells.

The methods for generating monoclonal antibodies (MAbs) generally begin along the same lines as those for preparing polyclonal antibodies. The use of rats may provide certain advantages (Goding, 1986), but mice are preferred, with the BALB/c mouse being the most routinely used and generally gives a higher percentage of stable fusions.

Following immunization, somatic cells with the potential for producing antibodies, specifically B lymphocytes (B cells), are selected for use in the MAb generating protocol. These cells may be obtained from biopsied spleens, tonsils or lymph nodes, or from a peripheral blood sample Spleen cells and peripheral blood cells are preferred. Often, a panel of animals will have been immunized and the spleen of animal with the highest antibody titer will be removed and obtaining lymphocytes from the spleen.

The antibody-producing B lymphocytes from the immunized animal are then fused with cells of an immortal myeloma cell, generally one of the same species as the animal that was immunized. Myeloma cell lines suited for use in hybridoma-producing fusion procedures preferably are non-antibody-producing, have high fusion efficiency, and have enzyme deficiencies that render them incapable of growing in certain selective media that support the growth of only the desired fused cells (hybridomas). Selected hybridomas are serially diluted and cloned into individual antibody-producing cell lines, which can then be propagated indefinitely to provide MAbs.

In accordance with the present invention, fragments of the monoclonal antibody useful in the invention can be obtained from the monoclonal antibody produced as described above, by methods which include digestion with enzymes such as pepsin or papain and/or cleavage of disulfide bonds by chemical reduction. Alternatively, monoclonal antibody fragments encompassed by the present invention can be synthesized using an automated synthesizer, or by expression of full-length gene or of gene fragments in E. coli or other recombinant microorganisms and cell lines.

Useful in the present invention are also various antibody conjugates. Conjugates with fluorescein markers are prepared be methods known in the art, such as conjugation in the presence of coupling agents or by reaction with an isothiocyanate. Conjugates with metal chelates are similarly produced. Other moieties to which antibodies may be conjugated include radionuclides such as ¹³¹I, ⁹⁰Y, ¹⁰⁵Rh, ⁴⁷Sc, ⁶⁷Cu, ²¹²Bi, ²¹¹At, ¹⁸⁸ Re, ¹⁰⁹Pd, ⁴⁷Sc, ²¹²Pb, and ¹⁵³Sm and the like, as described in Gansow, 1991.

Monoclonal antibodies can also be coupled to conventional chemotherapeutic agents such as an antimetabolite, an anthracycline, a vinca alkaloid, an antibiotic or an alkylating agent. Drugs that may be coupled to the antibodies for targeting include compounds such as doxorubicin, cyclophosphamide, cisplatin, adriamycin, estramustine, fluorouracil, ethinyl estradiol, mitoxantrone, methotrexate, finasteride, taxol, and megestrol. Methods of coupling may be direct via covalent bonds, or indirect via linking molecules, and will generally be known in the art for the particular drug selected and are made using a variety of bifunctional protein coupling agents. Examples of such reagents are SPDP, IT, bifunctional derivatives of imidoesters such a dimethyl adipimidate HCl, active esters such as disuccinimidyl suberate, aldehydes such as glutaraldehyde, bisazido compounds such as his (R-azidobenzoyl) hexanediamine, bisdiazonium derivatives such as bis-(R-diazoniumbenzoyl)ethylenediamine, diisocyanates such as tolylene 2,6-diisocyanate, and bis-active fluorine compounds such as 1,5-difluoro-2,4-dinitrobenzene. (*See, e*.*g*., Thorpe *et al.,* 1982.

The antibodies may also be conjugated with various toxin molecules or an effector such as IgGl immunoglobulin, which induces cytolytic or cytotoxic immune response. Thus, the two components may be chemically bonded together by any of a variety of well-known chemical procedures. For example, the linkage may be by way of heterobifunctional cross-linkers, e.g. SPDP, carbodiimide, glutaraldehyde, or the like. The toxin molecules may also be fused to the antibody or binding regions thereof by recombinant means, such as through the production of single chain antibodies. The genes encoding protein chains may be cloned in cDNA or in genomic form by any cloning procedure known to those skilled in the art (*see e.g.,* Sambrook *et al.,* 1989). The recombinant production of various immunotoxins is well-known within the art and can be found, for example in *Thorpe et al*., 1982(a), Waldmann, 1991, and Pastan *et al*., 1992. A variety of toxin molecules are suitable for use as the cytotoxic domain in the antibody conjugates or fusion proteins described here. Any toxin known to be useful as the toxic component of an immunotoxin may be used, preferably a protein toxin that may be recombinantly expressed. Particularly useful as the cytotoxic domain are bacterial toxins such as Pseudomonas exotoxin A (PE), diphtheria toxin, shiga toxin and shiga-like toxin, and ribosome inactivating toxins derived from plants and fungi, including ricin, α-sarcin, restrictotocin, mitogellin, tricanthosin, saporin-G, saporin-1, momordin, gelonin, pokeweed antiviral protein, abrin, modeccin and others described in Genetically Engineered Toxins, ed. A. Frankel, Marcel Dekker, Inc., 1992 and any recombinant derivatives of those proteins (see Olsnes 1981; U.S. Pat. No. 4,675,382; and U.S. Pat. No. 4,894,443).

The antibody may also be a bispecific antibody which recognizes both the MUC1/ECD and an antigen which promotes the release of a cytokine such as IL-1, TNF alpha and CD16, CD2, CD3 L.C. CD28, which in turn, will activate the release of IFN gamma or TNF alpha, respectively.

The MAb's useful in the present invention encompass chimeric Mabs, including, "humanized" forms of non-human (e.g., murine) Mabs. Humanized MAbs are chimeric antibodies which contain minimal sequence derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, framework residues of the human immunoglobulin are replaced by corresponding non-human residues. Furthermore, humanized antibodies may comprise residues which are not found in the recipient antibody or in the donor antibody. These modifications are made to further refine antibody performance. In general, the humanized antibody will comprise substantially all of at least one, and typically two, variable domains, in which all or substantially all of the hypervariable regions correspond to those of a non-human immunoglobulin and all or substantially all of the framework regions are those of a human immunoglobulin sequence. The humanized antibody optionally also will comprise at least a portion of an immunoglobulin constant region (Fc), typically that of a human immunoglobulin. (see Jones *et al.,* 1986; Riechmann *et al.,* 1988; and Presta, 1992). Fully human MAbs are preferred in the therapeutic methods of the present invention.

"Single-chain FV' or "sFv" antibody fragments of the present invention comprise the VH and VL domains of antibody, wherein these domains are present in a single polypeptide chain. Generally, the Fv polypeptide further comprises a polypeptide linker between the VH and VL domains which enables the sFv to form the desired structure for antigen binding (see Pluckthun, 1994).

### III. Screening and Diagnostic Assays

The present invention provides for methods for identifying compounds that inhibit the binding of various ligands to MUC1/ECD. The binding ligands include neuregulin 2 isoform 5 (SEQ ID NO: 2), neuregulin 2 isoform 6 (SEQ ID NO: 3) and fragments of either isoform that bind to MUC1/ECD and, in a preferred embodiment, an antibody that binds to an epitope within SEQ ID NO: 4.

In one embodiment, the screening method utilizes an *in vitro* competitive binding assay, wherein the capacity of a test compound to inhibit the binding of the aforementioned ligands to a polypeptide comprising SEQ ID NO.1 or SEQ ID NO: 5 is assessed. In such an assay, the polypeptide comprising MUC1/ECD derived sequences SEQ ID NO.1 or SEQ ID NO: 5 may be conjugated to another protein or produced as a fusion protein, *e.g.,* the GST-MUC1/ECD fusion protein exemplified herein in Example 3. Other suitable conjugates and fusion proteins may be made by one of skill in the art utilizing procedures know in the art. The polypeptides or MUC1/ECD ligands may be labeled with a radioisotope or fluorescent label (e.g., phycobiliproteins, such as phycoerythrin and allophycocyanins, fluorescein and Texas red). Alternatively an enzyme, such as peroxidase, may be used and conjugated either directly or indirectly via a biotin and avidin or streptavidin system. Decreased binding upon introduction of a test compound is indicative of competitive binding.

A compound that inhibits the binding of ligands to MUC1/ECD may be a modulator, that is an antagonist or agonist of the biological activity initiated by MUC1/ECD binding by neregulin 2 isoforms 5 or 6. *E.g*., the antibody raised to polypeptide P1 (SEQ ID. NO 4) is expected to inhibit binding of the wild-type ligands but acts as an agonist for the MUC1/ECD binding site, *i*.*e*., it stimulates proliferation of carcinoma cells. In contrast, appropriate compounds, such as the MUC1/ECD polypeptide SEQ ID NO. 1, will bind to the endogenous wild-type ligands thereby preventing binding to MUC1/ECD and consequently acting as an antagonist, *i.e.,* preventing or decreasing the proliferation of carcinoma cells that would be otherwise observed upon binding of the MUC1/ECD ligands.

An alternative screening assay can discriminate between MUC1/ECD binding inhibitors that exhibit antagonist and agonist activity in regard to the proliferation of MUC1-expressing cancer cells. The method requires a population of MUC1-positive cancer cells, preferably human cancer cells. This could be a population of cells that constitutively expresses MUC1, but the population is preferably of a cell type engineered to express NMC1. The latter are more versatile in regard to providing cells for appropriate controls, e.g. cells engineered with an empty vector, and also for enabling the construction of cells expressing MUC1 mutants. Examples of engineered MUC1 cancer cells include, but are not limited to, SW480 and HCT116 colon cancer cells as exemplified in Examples 2 and 4 herein. Inhibition of MUC1/ECD ligand-induced cell proliferation will indicate a test compound with antagonist activity. Controls may comprise incubation of cancer cells engineered with an empty vector (i.e. MUC1-negative) or incubation of MUC1-positive cells in the absence of either the test compound or the MUC1/ECD ligand. One of the latter controls will identify agonists, *i*.*e*., stimulation of cancer cell proliferation observed in incubations in which the test compound is present and the MUC1/ECD ligand is absent. Specificity of the agonist activity is established by use of engineered MUC1-negative cells.

Yet another screening assay monitors MUC1/ECD ligand induced phosphorylation of the intracellular domain of MUC1. Alternate screening methodologies employ monitoring of MUC1/ECD ligand induced association of MUC1 with EGF-R, s-Src, β-catenin, GSK3β or p120. Methods for monitoring such phosphorylation and protein associations are described in Li *et al*., (1998), Li *et al*., (2001), Li *et al*., (2001 (a)) and Li & Kufe, (2001).

The present invention also provides for methods for identifying compounds that downregulate the expression of MUC1/ECD. In some embodiments of the invention, labeled antibodies to MUC1/ECD are utilized to visualize the expression of MUC/ECD in appropriate cell lines by flow cytometry or by immunohistochemistry, using methods know in the art. Alternatively, the expression of MUC1 can be estimated by immunoblotting or by probing total cellular RNA with labeled DNA probes, *e.g.,* as described in Example 7 herein.

Estimation of the expression of MUC1/ECD can also be used for diagnostic methodologies, wherein antibodies to MUC1/ECD are utilized to investigated the expression of MUC1/ECD on or in cells derived from a subject. Such antibodies can also be utilized for imaging of cancer cells within a subject. Imaging is performed by labeling the anti-MUC1/ECD antibody, *e.g.,* with a radiolabel, and injecting the antibody to a subject and monitoring the location of the antibody within the body of said subject.

### IV. Combination with Chemotherapeutic Agents

The present invention encompasses the use of the MUC1/ECD antagonists in combination with chemotherapeutic agents for the manufacture of a medicament. While not being limited by any particular theory, MUC1 inhibits the apoptotic response to genotoxic stress induced by certain chemotherapeutic agents, and thereby induces resistance to such agents. MUC1/ECD antagonists may be used to mitigate this MUC1 mediated response to chemotherapeutic agents, thereby enhancing the effectiveness of such agents. In this regard, MUC1/ECD antagonists will be useful for the treatment cancer cells resistant to chemotherapeutic agents, including residual cancers remaining or reoccurring after cancer chemotherapy. The foregoing rational also pertains to the combination of MUC1/ECD antagonists and ionizing radiation

The chemotherapeutic agents useful in the medicament of the invention include the full spectrum of compositions and compounds which are known to be active in killing and/or inhibiting the growth of cancer cells. The chemotherapeutic agents, grouped by mechanism of action include DNA-interactive agents, antimetabolites, tubulin interactive agents, anti-hormonals, anti-virals, ODC inhibitors and other cytotoxics such as hydroxyurea. Any of these agents are suitable for use in the methods of the present invention.

DNA-interactive agents include the alkylating agents, *e.g.,* cisplatin, cyclophosphamide, altretamine; the DNA strand-breakage agents, such as bleomycin; the intercalating topoisomerase II inhibitors, *e.g.,* dactinomycin and doxorubicin); the nonintercalating topoisomerase II inhibitors such as, etoposide and teniposide; and the DNA minor groove binder plicamycin.

The alkylating agents form covalent chemical adducts with cellular DNA, RNA and protein molecules and with smaller amino acids, glutathione and similar chemicals. Generally, these alkylating agents react with a nucleophilic atom in a cellular constituent, such as an amino, carboxyl, phosphate, sulfhydryl group in nucleic acids, proteins, amino acids, or glutathione. The mechanism and the role of these alkylating agents in cancer therapy is not well understood. Typical alkylating agents include: nitrogen mustards, such as chlorambucil, cyclophosphamide, ifosfamide, mechlorethamine, melphalan, uracil mustard; aziridine such as thiotepa; methanesulphonate esters such as busulfan; nitroso ureas, such as carmustine, lomustine, streptozocin; platinum complexes such as cisplatin, carboplatin; bioreductive alkylators, such as mitomycin and procarbazine, dacarbazine and altretemine; DNA strand-breaking agents including bleomycin.

Topoisomerases are ubiquitous cellular enzymes which initiate transient DNA strand breaks during replication to allow for free rotation of the strands. The functionality of these enzymes is critical to the replication process of DNA. Without them, the torsional strain in the DNA helix prohibits free rotation, the DNA strands are unable to separate properly, and the cell eventually dies without dividing. Topo I links to the 3'-terminus of a DNA single strand break, while Topo II links to the 5'-terminus of a double strand DNA break. DNA topoisomerase II inhibitors include the following: intercalators such as amsacrine, dactinomycin, daunorubicin, doxoi-ubicin, idarubicin and mitoxantrone; nonintercalators such as etoposide and teniposide; camtothecins including irinotecan (CPT-II) and topotecan. A representative DNA minor groove binder is plicamycin.

The antimetabolites generally exert cytotoxic activity by interfering with the production of nucleic acids by one or the other of two major mechanisms. Some of the drugs inhibit production of the deoxyribonucleoside triphosphates that are the immediate precursours of DNA synthesis, thus inhibiting DNA replication. Some of the compounds are sufficiently like purines or pyrimidines to be able to substitute for them in the anabolic nucleotide pathways. These analogs can then be substituted into the DNA and RNA instead of their normal counterparts. The antimetabolites useful herein include: folate antagonists such as methotrexate and trimetrexate; pyrimidine antagonists such as fluorouracil, fluorodeoxyuridine, azacitidine, cytarabine, and floxuridine; purine antagonists include mercaptopurine, 6-thioguanine, fludarabine, pentostatin; sugar modified analogs include cytarabine, fludarabine; ribonucleotide reductase inhibitors include hydroxyurea.

Tubulin interactive agents interfere with cell division by binding to specific sites on Tubulin, a protein that polymerizes to form cellular microtubules. Microtubules are critical cell structure units. When the interactive agents bind on the protein, the cell cannot properly form microtubules. Tubulin interactive agents include vincristine and vinblastine, both alkaloids and the taxanes (paclitaxel and docetaxel).

Although their mechanisms of action are different, both taxanes and vinca alkaloids exert their biological effects on the cell microtubles. Taxanes act to promote the polymerization of tubulin, a protein subunit of spindle microtubles. The end result is the inhibition of depolymerization of the microtubles, which causes the formation of stable and nonfunctional microtubles. This disrupts the dynamic equilibrium within the microtuble system, and arrests the cell cycle in the late G₂ and M phases, which inhibits cell replication.

Like taxanes, vinca alkaloids also act to affect the microtuble system within the cells. In contrast to taxanes, vinca alkaloids bind to tubulin and inhibit or prevent the polymerization of tubulin subunits into microtubles. Vinca alkaloids also induce the depolymerization of microtubles, which inhibits microtuble assembly and mediates cellular metaphase arrest. Vinca alkaloids also exert effects on nucleic acid and protein synthesis; amino acid, cyclic AMP, and glutathione synthesis; cellular respiration; and exert immunosuppressive activity at higher concentrations.

Antihormonal agents exert cytotoxic activity by blocking hormone action at the end-receptor organ. Several different types of neoplasm require hormonal stimulation to propagate cell reproduction. The antihormonal agents, by blocking hormone action, deprive the neoplastic cells of a necessary stimulus to reproduce. As the cells reach the end of their life cycle, they die normally, without dividing and producing additional malignant cells. Antihormonal agents are typically derived from natural sources and include: estrogens, conjugated estrogens and ethinyl estradiol and diethylstibesterol, chlortrianisen and idenestrol; progestins such as hydroxyprogesterone caproate, medroxyprogesterone, and megestrol; androgens such as testosterone, testosterone propionate; fluoxymesterone, methyltestosterone.

Adrenal corticosteroids are derived from natural adrenal cortisol or hydrocortisone. They are used because of their anti-inflammatory benefits as well as the ability of some to inhibit mitotic divisions and to halt DNA synthesis, these compounds include prednisone, dexamethasone, methylprednisolone, and prednisolone. Leutinizing-releasing hormone agents or gonadotropin-releasing hormone antagonists are used primarily in the treatment of prostate cancer. These include leuprolide acetate and goserelin acetate. They prevent the biosynthesis of steroids in the testes.

Anti-hormonal agents include antiestrogenic agents such as tamoxifen, antiandrogen agents such as flutamide, and antiadrenal agents such as mitotane and aminoglutethimide.

ODC (or ornithine decarboxylase) inhibitors inhibit cancerous and pre-cancerous cell proliferation by depleting or otherwise interfering with the activity of ODC, the rate limiting enzyme of polyamine biosynthesis important to neoplastic cell growth. In particular, polyamine biosynthesis wherein ornithine is converted to the polyamine, putrescine, with putrescine being subsequently by converted to spermidine and spermine appears to be an essential biochemical event in the proliferation of neoplastic growth in a variety of cancers and cancer cell lines and the inhibition of ODC activity or depletion of ODC in such neoplastic cells has been shown to reduce polyamine levels in such cells leading to cell growth arrest; more differentiated cell morphology and even cellular senescence and death. In this regard, ODC or polyamine synthesis inhibitors are considered to be more cytotoxic agents functioning to prevent cancer reoccurrence or the conversion of pre-cancerous cells to cancerous cells than cytotoxic or cell killing agents. A suitable ODC inhibitor is eflornithine or α-difluoromethyl-ornithine, an orally available, irreversible ODC inhibitor, as well as a variety of polyamine analogs which are in various stages of pre-clinical and clinical research.

Other cytotoxics include agents which interfere or block various cellular processes essential for maintenance of cellular functions or cell mitosis as well as agents which promote apoptosis. In this regard, hydroxyurea appears to act via inhibitors of the enzyme ribonucleotide reductase whereas asparaginase enzymatically converts asparagine into non-functional aspartic acid thereby blocking protein synthesis in a tumor.

Compositions of the MUC1/ECD antagonists of present invention can also be used in combination with antibodies to HER-2, such as Trastuzumab (Herceptin (H)). In addition, the present invention also emcompasses the use of MUC1 domain antoagonists in combination with epidermal growth factor recpetor-interactive agents such as tyrosine kinase inhibitors. Tyrosine kinase inhibitors suitably include imatinib (Norvartis), OSI-774 (OSI Pharmaceuticals), ZD-1839 (AstraZeneca), SU-101 (Sugen) and CP-701 (Cephalon).

When used in the present invention, it is contemplated that the chemotherapeutic agent of choice can be conveniently used in any formulation which is currently commercially available, and at dosages which fall below or within the approved label usage for single agent use.

### V. Ionizing Radiation

In the present invention, the term "ionizing radiation" means radiation comprising particles or photons that have sufficient energy or can produce sufficient energy via nuclear interactions to produce ionization (gain or loss of electrons). An exemplary and preferred ionizing radiation is an x-radiation. Means for delivering x-radiation to a target tissue or cell are well known in the art. The amount of ionizing radiation needed in a given cell generally depends on the nature of that cell. Means for determining an effective amount of radiation are well known in the art. Used herein, the term "an effective dose" of ionizing radiation means a dose of ionizing radiation that produces cell damage or death when given in conjunction with the MUC1/ECD antagonists useful in the present invention, optionally further combined with a chemotherapeutic agent.

Dosage ranges for x-rays range from daily doses of 50 to 200 roentgens for prolonged periods of time (3 to 4 weeks), to single doses of 2000 to 6000 roentgens. Dosage ranges for radioisotopes vary widely, and depend on the half-life of the isotope, the strength and type of radiation emitted, and the uptake by the neoplastic cells.

Any suitable means for delivering radiation to a tissue may be employed in the present invention, in addition to external means. For example, radiation may be delivered by first providing a radiolabeled antibody that immunoreacts with an antigen of the tumor, followed by delivering an effective amount of the radiolabeled antibody to the tumor. In addition, radioisotopes may be used to deliver ionizing radiation to a tissue or cell.

### VI. Downregulation of MUC1/ECD Expression

Some compounds downregulate MUC1/ECD expression. One such compound is the isocoumarin NM-3 (2-(8-hydroxy-6-methoxy-1-oxo-1*H*-2-benzopyran-3-yl) propionic acid). NM-3 and other isocoumarins suitable to downregulate the expression of MUC1/ECD are disclosed in U.S. patent No: 6,020,363.

Other suitable compounds include 2-substituted estradiol compounds such as 2-methoxyestradiol and 2-hydroxyrestradiol. These and other suitable estradiol derivatives are disclosed in U.S. Patent No. 6,239,123. Other compounds suitable for downregulating MUC1/BCD expression include antisense oilgonucleotides that target nucleic acid molecules encoding MUC1, as described below.

### VII. Antisense Oligonucleotides

Antisense compounds, particularly oilgonucleotides, for use in modulating the function of nucleic acid molecules encoding MUC1 and MUC1/ECD wild-type ligands, such as neureguiin 2 isoforms 5 and 6 can also be used. Inhibition of MUC1 expression will decrease the levels of MUC1/ECD available for binding to MLTC1/ECD ligands. Inhibition of the expression of the endogenous ligands of MUC1/ECD will prevent or decrease the proliferative effect on cancer cells associated with the binding of such ligands to MUC1/ECD. Antisense methodology takes advantage of the fact that nucleic acids tend to pair with "complementary sequences." By complementary, it is meant that polynucleotides are those capable of base-pairing according to the standard Watson-Crick complementary rules. The oligonucleotides may be targeted wholly or in part to informational sequences, *i*.*e*., those coding for a protein, and other associated ribonucleotides such 5'-untranslated regions, 3'-untranslated regions, 5' cap regions and intron/exon junctions. The overall effect of interference with mRNA is modulation of expression of neuregulin isoforms 5 and/or 6. Such modulation can be measured in ways that are routine in the art. In addition, effects on cancer cell proliferation or tumor growth can be assessed.

It is understood that an oligonucleotide need not be 100% complementary to its target nucleic acid sequence to be specifically hybridizable. An oligonucleotide is specifically hybridizable when binding of the oligonucleotide to the target interferes with the normal function of the target molecule to cause a loss of utility, and there is a sufficient degree of complementarity to avoid non-specific binding of the oligonucleotide to non-target sequences under conditions in which specific binding is desired, i.e., under physiological conditions in the case of in vivo assays or therapeutic treatment.

The antisense compounds preferably comprise from about 4 to about 50 nucleobases. Particularly preferred are antisense oligonucleotides comprising from about 8 to about 30 linked nucleobases. The oligonucleotides used may be conveniently and routinely made through the well-known technique of solid phase synthesis. In the context of this invention, the term "oligonucleotide" refers to an oligomer or polymer of ribonucleic acid or deoxyribonucleic acid. This term includes oligonucleotides composed of naturally-occumng nucleobases, sugars and covalent intersugar (backbone) linkages as well as oligonucleotides having non-naturally-occurring portions which function similarly. Such modified or substituted oligonucleotides are often preferred over native forms because of desirable properties such as, for example, enhanced cellular uptake, enhanced binding to target and increased stability in the presence of nucleases. Examples of some preferred modified oligonucleotides include those containing phosphorothioates, phosphotriesters, methyl phosphonates, short chain alkyl or cycloalkyl intersugar linkages or short chain heteroatomic or heterocyclic intersugar linkages.

Another additional or alternative modification of the oligonucleotides involves chemically linking to the oligonucleotide one or more lipophilic moieties which enhance the cellular uptake of the oligonucleotide. Such lipophilic moieties may be linked to an oligonucleotide at several different positions on the oligonucleotide. Some preferred positions include the 3' position of the sugar of the 3' terminal nucleotides, the 5' position of the sugar of the 5' terminal nucleotide, and the 2' position of the sugar of any nucleotide. The antisense compounds also include bioequivalent compounds, including pharmaceutically acceptable salts and prodrugs.

The terms "specifically hybridizable" and "complementary" are used to indicate a degree of complementarity sufficient to result in stable and specific binding between the antisense oligonucleotide and the target nucleic acid sequence. An oligonucleotide need not be 100% complementary to its target nucleic acid sequence to be specifically hybridizable. An oligonucleotide considered "specifically hybridizable" when binding of the oligonucleotide to the target interferes with the normal function of the target molecule to cause a loss of utility and decrease in expression of the product protein, and there is a sufficient degree of complementarity to avoid non-specific binding of the oligonucleotide to non-target sequences.

The neuregulin 2 protein family comprises a number of alternatively spliced isoforms (Ring *et al.,* 1999). The coding sequences for neuregulin 2 isoforms 5 and 6 share the same nucleotide sequence from exons 1 through 6 of the neuregulin 2 gene that code for the first 416 amino acids of each protein but differ in the sequence coding for the carboxy terminal 10 amino acids of isoform 5 and for the carboxy terminal 6 amino acids of isoform 6. The coding DNA sequences of exons 1 through 6 are incorporated in SEQ ID NO: 10 through SED ID. NO: 15 respectively. The sequences coding for the first 416 amino acids of isoforms 5 and 6 are nucleotides 313 through 1012 of SEQ ID. NO 10, nucleotides 51-222 of SEQ ID. NO: 11, nucleotides 230-348 of SEQ ID NO: 12, nucleotides 100 through 220 of SEQ ID. NO: 13, nucleotides 111 through 187 of SEQ ID. NO: 14 and nucleotides 123 through 181 of SEQ In. NO: 15. The sequences coding for the carboxy terminals of isoform 5 and isoform 6 are nucleotides 132 through 164 of SEQ ID NO: 16 and nucleotides 30 through 50 of SEQ ID NO: 17 respectively.

As SEQ ID NO: 16 and SEQ ID NO: 17 are apparently not shared by other neuregulin gene products, in a preferred embodiment, the antisense oligonucleotide comprises a sequence of at least 4 nucleotides that is complementary to a region between nucleotides 132 and 164 of SEQ ID. NO: 16 or nucleotides 30 through 50 of SEQ ID NO: 17. In a more preferred embodiment, the antisense oilgonucleotides comprises a sequence of at least 8 nucleotides that is complementary to a region between nucleotides 132 and 164 of SEQ ID. NO: 16 or nucleotides 30 through 50 of SEQ ID NO: 17.

In other embodiments, the antisense oligonucleotide comprises a sequence of at least 4 nucleotides that is complementary to a region between nucleotides 313 through 1012 of SEQ ID. NO 10, or a region between nucleotides 51-222 of SEQ ID. NO: 11, or a region between nucleotides 230-348 of SEQ ID NO: 12, or a region between nucleotides 100 through 220 of SEQ ID. NO: 13, or a region between nucleotides 111 through 187 of SEQ ID. NO: 14, or a region between nucleotides 123 through 181 of SEQ ID. NO: 15. In another embodiment the antisense oligonucleotide is at least 8 nucleotides that is complementary to a region of the one the foregoing nucleotides sequences.

In other embodiments, the antisense oligonucleotide comprises a sequence of at least 4 nucleotides, and preferably a sequence of at leas 8 nucleotides, that is complementary to a non-coding region of SED ID NOS: 10 through 17.

In other embodiments, MUC1 directed antisense oligonucleotides comprise a sequence of at least 4 nucleotides that is complementary to SEQ ID NO: 18. In preferred.embodiments, the antisense oligonucleotides comprises a sequence of at least 8 nucleotides that is complementary to SEQ ID NO: 18

Expression vectors comprising an expression control system that directs production of a transcript of the foregoing antisense oilgonucleotides can also be used. In addition, methods of hybridization comprising providing one of the forgoing antisense oilgonucleotides and contacting such oligonucleotide with a nucleic acid comprising the target sequence under conditions that permit hybridization of the oligonucleotide with the nucleic acid can be applied. Also included are methods of inhibiting translation of mRNA comprising providing one of the forgoing antisense oilgonucleotides and providing a cell comprising mRNA comprising the target sequence and introducing the oligonucleotide into the cell, wherein the oligonucleotide inhibits translation of the mRNA in the cell.

Pharmaceutical compositions comprising an antisense oligonucleotide of the invention and a pharmaceutically acceptable carrier, can also be applied.

### VIII. Vaccines

The present invention also encompasses the use of MUC1/ECD peptides, *e.g*,. SEQ ID NO: 1 or fragments thereof, wherein such fragments comprise four or more consecutive amino acids of SEQ ID NO. 1, in a vaccine wherein the host mammal generates antibodies to the polypeptide which also act against the host's own MUC1/ECD. Vaccine preparation techniques are generally known in the art as described by Duffy (1980), and references cited therein.

The MUC1/ECD peptides may be conjugated to a carrier molecule such as a protein or Ficoll. The carrier protein is preferably one with a molecular weight of at least about 40,000 dalton and more preferably at least about 60,000 dalton. The vaccine formulation may comprise a pharmaceutically acceptable carrier and may also include adjuvant systems for enhancing the immunogenicity of the formulation, such as oil-in water systems and other systems known in the art. Since the peptides or conjugates may be broken down in the stomach, the vaccine is preferably administered parenterally (for instance, subcutaneous, intramuscular, intravenous, or intradermal injection). The dosage will depend on the specific activity of the vaccine and can be readily determined by routine experimentation. The formulations may be presented in unit-dose or multi-dose containers, for example, sealed ampoules and vials and may be stored in a freeze-dried condition requiring only the addition of the sterile liquid carrier immediately prior to use.

### IX. Formulations

The MUC1/ECD antagonists including binding inhibitors employed in the compositions and methods of the present invention can be formulated in a variety of conventional pharmaceutical formulations and administered to cancer patients, in need of treatment, by any one of the drug administration routes conventionally employed including oral, intravenous, intraarterial, parental or intrapenitoneal.

For oral administration the compositions of the present invention may be formulated, for example, with an inert dilutent or with an assimiable edible carrier, or enclosed in hard or soft shell gelatin capsules, or compressed into tablets, or incorporated directly with the food of the diet. For oral therapeutic administration, the active compound may be incorporated with excipients and used in the form of ingestible tablets, buccal tablets, troches, capsules, elixirs, suspensions, syrups, wafers, and the like. Such compositions and preparations may, of course, be varied and may conveniently be between about 2 to about 60% of the weight of the unit. The amount of active compounds in such therapeutically useful compositions is such that a suitable dosage will be obtained.

The tablets, troches, pills, capsules and the like may also contain the following: a binder, a gum tragacanth, acacia, cornstarch, or gelatin; excipients, such as dicalcium phosphate; a disintegrating agent, such as corn starch, potato starch, alginic acid and the like; a lubricant, such as magnesium stearate; and a sweetening agent, such as sucrose, lactose or saccharin may be added or a flavoring agent, such as peppermint, oil of wintergreen, or cherry flavoring. When the dosage unit for is a capsule, it may contain, in addition to materials of the above type, a liquid carrier. Various other materials may be present as coatings or to otherwise modify the physical form of the dosage unit. For instance, tablets, pills, or capsules may be coated with shellac, sugar or both. A syrup or elixir may contain the active compounds sucrose as a sweetening agent methyl and propylparabens as preservatives, a dye and flavoring, such as cherry or orange flavor. Of course, any material used in preparing a dosage unit form should be pharmaceutically pure and substantially non-toxic in the amounts employed. In addition, other chemotherapeutic compounds may be incorporated into sustained-release preparation and formulations.

In regard to formulations comprising oligonucleotides, colloidal dispersion systems may be used as delivery vehicles to enhance the *in vivo* stability of the oligonucleotides and/or to target the oligonucleotides to a particular organ, tissue or cell type. Colloidal dispersion systems include, but are not limited to, macromolecule complexes, nanocapsules, microspheres, beads and lipid-based systems including oil-in-water emulsions, micelles, mixed micelles, liposomes and lipid:oligonucleotide complexes of uncharacterized structure.

Pharmaceutical formulations of the compositions of the present invention which are suitable for injectable use include sterile aqueous solutions or dispersions and sterile powders for the extemporaneous preparation of sterile injectable solutions or dispersions. In all cases the form must be sterile and must be fluid to the extent that each syringability exists. It must be stable under the conditions of manufacture and storage and must be preserved against the contaminating action of microorganisms, such as bacteria and fungi. The carrier can be a solvent or dispersion medium containing, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the compositions of the present invention in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

As used herein, "pharmaceutically acceptable carrier" includes any and all solvents, dispersion media, coatings, antibacterial and antifungal agents, isotonic and absorption delaying agents and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the composition.

### X. Treatment Methods

Tumors that can be suitably treated with the medicament of the present invention include; but are not limited to, tumors of the brain (glioblastomas, medulloblastoma, astrocytoma, oligodendroglioma, ependymomas), lung, liver, spleen, kidney, lymph node, small intestine, pancreas, blood cells, colon, stomach, breast, endometrium, prostate, testicle, ovary, skin, head and neck, esophagus, bone marrow, blood and other tissue. The tumor may be distinguished as metastatic and non-metastatic: Pre-malignant lesions may also be suitably treated with the medicament of the present invention.

The treatment, with the MUC1/ECD antagonists may precede or follow irradiation and/or chemotherapy by intervals ranging from seconds to weeks and/or be administered concurrently with such treatments. In embodiments where the MUC1/ECD antagonists and irradiation and/or chemotherapy are applied separately to the cell, steps should be taken to ensure that a significant period of time does not expire between the time of each delivery, such that the combination of the two or three treatments would still be able to exert an advantageously combined effect on the cell. In such instances, it is contemplated that one would contact the cell with the treatment agents or modalities within amount 0.1 to 25 h of each other and, more preferably, within about 1 to 4 h of each other, with a delay time of only about 1 h to about 2 h being most preferred. In some situations, it is desirable to extend the time period of treatment significantly, however, where several days (2, 3, 4, 5, 6 or 7) or several weeks (1, 2, 3, 4, 5, 6, 7 or 8) lapse between the respective administrations. In any case, the invention contemplates that the MUC1/ECD antagonist comprising medicament may be given before, after or even simultaneously with the ionizing radiation and/or chemotherapeutic agent.

Some chemotherapeutic agents transiently induce MUC1 expression on cancer cells 0.5 to 12 hours after contact of the carcinoma cells with the chemotherapeutic agents. Thus, in some embodiments the administration of MUC1/ECD binding inhibitors, especially antibodies to the MUC/ECD sequence SEQ ID NO. 1, optionally conjugated to a toxin or radionucleotide, is coordinated with the increased expression of MUC1 on the cancer cells. In other embodiments, agents other than chemotherapeutic agents may be used to increase MUC1 expression prior to treatment with a MUC1 binding inhibitor, especially antibodies to the MUC/ECD sequence SEQ ID NO. 1, optionally conjugated to a toxin or radionucleotide.

In the medicament of the present invention, the actual dosage of MUC1/ECD antagonists employed will depend on a variety of factors including the type and severity of cancer being treated, and the additive or synergistic treatment effects of the MUC1/ECD antagonists and the other treatment modality or modalities selected.

### EXAMPLES OF THE INVENTION

### Example 1: Peptides

The MUC1/ECD polypeptide sequence, as typically found in MUC1-expressing cells, is provided by SEQ ID NO: 1. A number of polypeptide sequences have been synthesized by standard techniques. These include peptides P1 (SEQ ID NO:4), P2 (SEQ ID NO: 5) and P3 (SEQ ID NO:6), which are polypeptide fragments of MUC1/ECD. P1 (SEQ ID NO : 4) represents amino acids 5 through 20 of MUC1/ECD (SEQ ID NO: 1) with a cysteine added at the carboxy terminal. P2 (SEQ ID NO: 5) represents amino acids 13 through 28 of MUC1/ECD (SEQ ID NO: 1) with a cysteine added at the carboxy terminal. P3 (SEQ ID NO: 6) represents amino acids 27 through 44 of MUC1/ECD (SEQ ID NO: 1) with a cysteine at the carboxy terminal. In addition, the synthesized polypeptide sequence SEQ ID NO: 7 incorporates amino acids 6 through 24 of MUC1/ECD (SEQ ID NO: 1) with a cysteine added at the carboxy terminal. The synthesized polypeptide P0 (SEQ ID NO: 8) incorporates a 19 amino acid sequence occurring in the MUC1 protein occurring just prior to the amino terminus of MUC1/ECD and represents the a potential cleavage site. A cysteine was again added at the carboxy terminal of the sequence at it occurs in the MUC1 sequence.

### Example 2: Anti-MUC1-P1 Antibody

### A. Generation of Antibody

The polypeptide P1 (SEQ. ID NO: 4), contains the QYK motif and other sequences homologous to ligand binding domains of cytokine receptors (Zrihan-Licht, *et al*., 1994). A polyclonal antibody was raised in rabbits against polypeptide P1 (SEQ ID NO. 4) conjugated to KLH. Serum was prepared by standard methods.

Polyclonal antibodies were also raised against the polypeptide SEQ ID NO: 7, whereby the immunogen was formed by conjugating the polypeptide SEQ ID NO: 7 to KLH. Antibodies have been obtained from 2 rabbits, designated 3402-1 and 3402-2. Both serum and affinity purified antibody preparations have been prepared by standard methodologies.

### B. Stimulation of Human Carcinoma Cells by Anti-MUC1-P1-Antibody

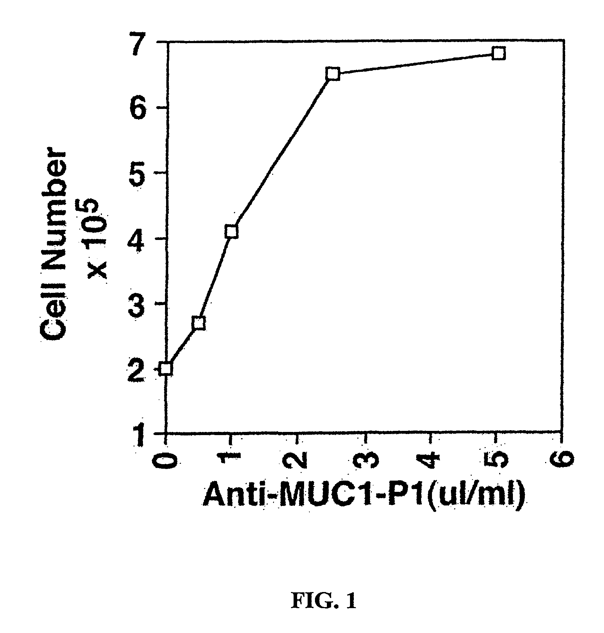

Human ZR-75-1 carcinoma cells were grown to 80% confluence in RPMI 1640 medium containing 10% fetal bovine serum (FBS) and then passed onto a 6-well plate at 1x10⁴ cells per well. After overnight starvation in medium containing 0.1% FBS, anti-MUC1-P1 antibody was added to each well it the amounts indicated in FIG. 1 and incubated for 48 hours. Cell numbers were quantified after another 3 days of incubation in the presence of 0.1 % FBS. As shown in FIG. 1, anti-MUCi-P1 stimulates the growth of ZR-75-1 cells in a dose dependent fashion.

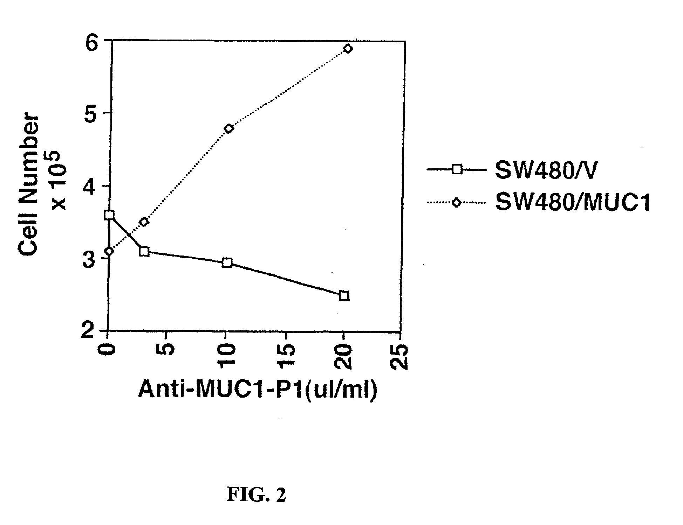

To assess the specificity of anti-MUC1-P1 antibody stimulation, human MUC1-negative SW480 colon cancer cells were stably transfected to express empty vector (SW480/V) or MUC1 (SW480/MLTC1). SW80 colon cancer cells were transfected with either pCMV-DE-ak1-dhfr vector or pCMV-IE-ak1-dhfr-MUC1 using lipofectamide (Ligtenburger *et al*., 1992). Cells were grown in the presence of 800 µg/ml G418 (neomycin) and serially diluted to single-cell populations. Single cell clones that express MUC1 (SW480/MUC1) were selected. Both SW480 cells types were grown to 80 % confluence in DMEM containing 10% FBS and plated onto 6-well plates at 5x10⁴ cells per well. After overnight starvation in medium containing 0.1% FBS, anti-MUC1-P1 antibody was added at the indicated concentrations and incubated for 48 hr. Cell numbers were quantified after a further day of incubation (3 days total). As shown in FIG. 2, anti-MUC1-P1 stimulates the growth of SW480/MUC1 cells but not SW48-0/V cells. These findings confirm that the anti-MUC1-P1 antibody stimulates the growth of human carcinoma cells by specifically interacting with MUC1.

### Example 3: Endogenous MUC1 ECD Ligands

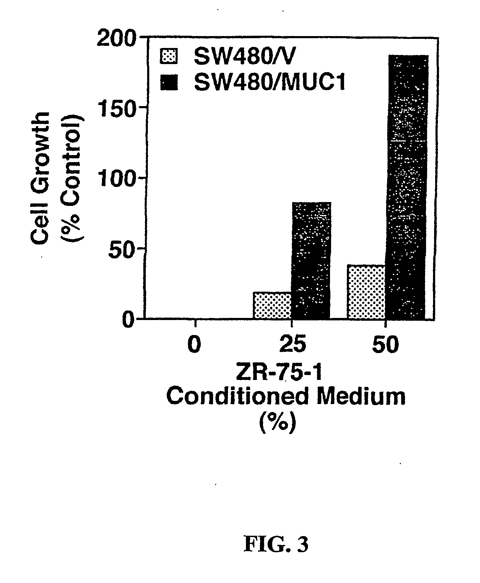

The finding that anti-MUC1-P1 stimulates growth of human carcinoma cells is suggestive of the potential existence of natural MUC1 ECD ligands. To investigate this potential, ZR-75-1 cells were screened as a possible source of MUC1 ligand(s). Conditioned medium was prepared by culturing ZR-75-1 cells in RPMI 1640 medium containing 0.1% FBS for 72 hr and then preparing a supernatant. The conditioned medium was added to SW480/V and SW480/MUC1 cells that were growth arrested in DME containing 0.1% FBS as previously described. Conditioned medium was added at the concentration indicated in Fig. 3 and the cells were maintained for 3 days prior to quantification of cell numbers. As shown in FIG. 3, ZR-75-1 cells express a soluble ligand that stimulates carcinoma cell growth by binding to MUC1.

To identify the soluble MUC1 ligand(s), a fusion protein comprising the MUC1/ECD (SEQ ID. No: 1) and glutathione S-transferase (GST) was prepared. GST was amplified by PCR using a set of primers as follows:
5'-ATTAGGCTAGCCTGGTTCCGCGTGGTTCTATGTCCCCTATACTAGGTTA-3',
   and 5'-CAAGGGGATCCCTACGGAACCAGATCCGATTTTGG-3', and inserted between the Nhe1 and BamH1 sites of pET-11d (the "pET-11d-GST vector"). MUC1/ECD was amplified by PCR using a set of primers as follows:
5'-TCTGGCCATGGGAGAAGGTACCATCAAT-3', and
5'-AGCGCGCTAGCCCAGCCTGGCACCCCAGC-3', and inserted between the Nco1 and Nhe1 sites of pET11d-GST vector (the "pET11d-GST-MUC1/ECD" vector).

The GST fusion protein was prepared by incubating logarithmically growing E. coli BL21(D3)pLysS cells transformed with pET11d-GST-MUC1/ECD or pET11d-GST with 0.1 mM isopropyl-β-D-thiogalatopyranoside for 6 hr at 25 °C. Cells were pelleted and resuspended in PBS containing 20 % sucrose, 5 mM MgCl₂, 0.5 % NP-40, then sonicated. Debris was removed by centrifugation at 10,000 x g for 30 min. at 4°C. The supernatant was applied to bulk glutathione sepharose 4B and incubated for 4 hr at 4 °C prior to washing and packing into a column. The fusion protein was eluted with 10 mM glutathione in 50 mM Tris-HCl, pH 9.5. The purified fusion protein was dialyzed with PBS.

Cytosolic fractions of cultured cells were prepared by harvesting ZR75-1 cells in PBS containing 40mM EDTA. After washing with PBS, cells were resuspended with ice-cold homogenized buffer (20 mM Hepes-KOH, pH 7.5, 10 mM KCl, 1.5 mM MgCl₂, 1 mM dithiothreitol, 1 mM EGTA, 1 mM EDTA and protease inhibitors cocktail (Roche) and put on ice for 15 min. The suspension was homogenized with a Dounce homogenizer. The homogenate was centrifuged at 1,00 x g for 5 min at 4 °C. The supernatant was collected as a cytosol fraction and stored at 0 °C.

The GST-MUC1/ECD fusion protein (1.8 mg) was immobilized on 400 µl of glutathione-sepharose 4B, which was packed into a column and equilibrated with buffer A (30 mM Tris-HCl, pH 7.5, 5 mM MgCl₂, 1 mM EDTA, and 1 mM dithiothreitol). The cytosolic fraction was first precleared by passing it through a glutathione-sepharose 4B column and was then loaded onto the GST-MUC1/ECD affinity column which was then washed twice with 2 x 10 ml of buffer A. The protein bound to the column was eluted by the addition of 2 ml of buffer B (buffer A containing 0.15 M NaCl), and fractions of 0.4 ml each were collected. The second and third fractions were mixed and loaded on sodium dodecyl sulfate-polyacyralmide gel electrophoresis (SDS-PAGE). As a control, the GST protein (1.5 mg) was immobilized on 400 µl of glutathione-sepharose 4B and the experiment performed as described above. The results demonstrated that NWC1/ECD binds to a 45 kDa protein. A similar experiment was performed with lysates of human MCF-7 breast carcinoma cells confirmed the binding of MUC1/ECD with a 45 kDa protein.

The 45 kDa from the GST-MUC1/ECD adsorbate was excised from the gel, dehydrated with acetonitrile, and then redhydrated with 100 mM ammonium bicarbonate. The gel pieces were then suspended in 12.5 ng/µl trypsin/50 mM ammonium bicarbonate. Digestion was carried out at 37 °C for 10-12 hr. The masses of the trypsin-digested peptides were analyzed by matrix assisted laser desorption/ionization-time of flight-mass spectroscopy (MALDI-TOF-MS) using a Voyager DE-PRO (Perceptive Biosystem Inc., Framingham, MA). Two related proteins, designated ML-1 and ML-2, were identified by mass fingerprinting. The sequences of ML-1 (SEQ ID NO:2 and ML-2 (SEQ ID NO:3) are as those previously disclosed for two neuregulin isoforms, NRG2 splice isoform 5 and NRG2 splice isoform 6 respectively.

### Example 4: MUC1 as an Oncogene

### A. MUC1 supports growth in soft agar

The expression of MUC1 was shown to be functionally significant regarding the exhibition of the malignant phenotype. Cell lines that stably express MUC1 were generated. HCT116 colon cancer cells were transfected with pIRES-puro2 vector or pIRES-puro2-MUC1 by lipofectamine and selected for puromycin-resistance. Studies were performed with human MUC1-negative HCT116 colon cancer cells with single clones that stably express either the empty vector (VCT116/V) or MUC1 (HCT116/MUC1). The HCT 116/V and HCT116/MUC1 cells were assayed for anchorage-independent growth in soft agar. Cells (1 x 10⁵ / 60 mm dish) were suspended in 0.33% agarose-containing DMEM medium supplemented with 10 % FBS and layered over an agarose plug (0.5 % agarose in DMEM supplemented with 10 % FBS). The cells were incubated to 4 weeks, during which time fresh medium was added to the plates every week. Colonies larger than 70 µm in diameter were counted after 4 weeks. Expression of wild-type MUC1 was associated with a marked increase in the size and number of colonies compared to that obtained with HCT/116/V cells. These findings were confirmed by similar studies performed with SW480/V and SW480/MUC1 cells wherein MUC1 expression was again shown to support anchorage-independent growth of SW480 cells.

### B. MUC1 supports human tumor formation in nude mice

To assess the effects of MUC1 on human tumor growth in vivo. Five to six week old athymic, Balbc/nu/nu mice (Taconic, Germantown, NY) were injected subcutaneously in the right flank with 1 x 10⁶ HCT116/V or HCT 116/MUC1 cells. Tumors (4 mice/group) were measured twice a week. Tumor volumes were calculated by the following formula: ½(length x width²). Experiments were terminated when tumor volume exceeded 2 cm³. Measurements of tumor volume over time demonstrated little HCT116/V cell growth. By comparison there was a marked increase in the growth of HCT116/MUC1 tumors.

### Example 5: MUC1 Expression is Induced by Oxidative and Genotoxic Stress

To determine whether MUC1 is induced in response to oxidative stress, MCF-7 cells were treated with hydrogen peroxide. Cell lysates were analyzed by immunoblotting with anti-DF3/MUC1 antibody. Lysates were prepared by suspending MCF-7 cells in lysis buffer (50 mM Tris, pH 7.6, 150 mM NaCl, 1 mM PMSF, 3 mM NaF, 1 mM sodium vandate, 1mM DTT with protease inhibitors and either 1% NP-40 or 1% Brij-96) for 30 min. on ice. Lysates were cleared by centifugation and equal amounts of proteins were resolved by SDS-PAGE. Proteins were then transferred to nitrocellulose filters, blocked by incubation in 5 % non-fat dry milk in PBS with 0.05 % Tween-20 and probed with anti-MUC1 antibody (Pandey *et al*., 1995). Anti-actin was used as a control. The results demonstrated that MUC1 is rapidly and transiently induced in response to oxidative stress while there was no effect of hydrogen peroxide on levels of actin expression.

Similar studies were performed with genotoxic agents. The results demonstrate that treatment of MCF-7 cells with daunorubicin is associated with a rapid and transient induction of MUC1, and not actin, expression. The available evidence indicates that MUC1 is induced by diverse cytotoxic agents, including taxol, cisplatin and ionizing radiation.

### Example 6: MUC1 Inhibits the Apoptotic Response to Oxidative and Genotoxic Stress

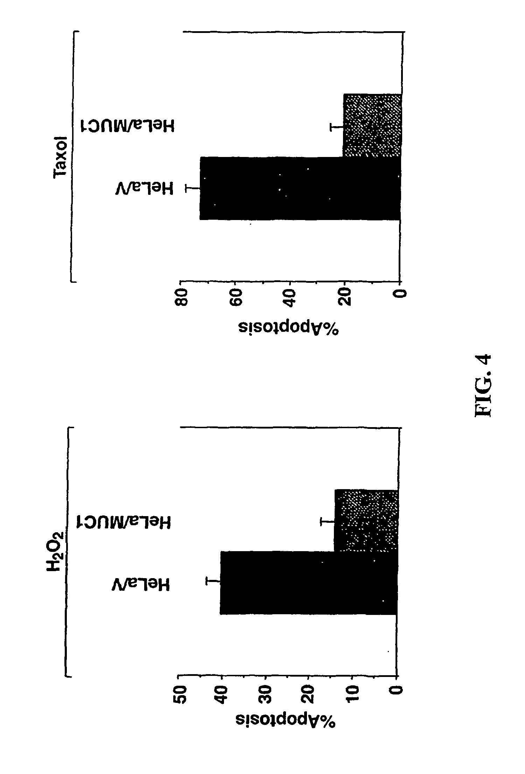

To determine whether MUC1 inhibits the apoptotic response to oxidative stress, HeLa cells stably expressing the empty vector or MUC1 were treated with 1 mM hydrogen peroxide for 1 hr. DNA content was assessed by staining ethanol-fixed cells with propidium iodide and monitoring by FACScan (Beckton Dickerson). Numbers of cells with sub-G1 DNA content were determined with a MODFIT LT program, (Verity Software House, Topsham, ME) (Yuan *et al.,* 1997). The cells were analyzed for induction of sub-G1 DNA by flow cytometry as a marker of apoptosis. As shown in FIG. 4, the results demonstrate that MUC1 inhibits the apoptotic response to oxidative stress.

The induction of apoptosis in cells treated with 0.01 mM taxol for 20 hr was assessed by measuring sub-G1 DNA content of HeLa cells expressing empty vector or MUC1. As shown in FIG. 4, as with oxidative stress, taxol-induced apoptosis was inhibited by MUC1 expression.

### Example 7: Effect of NM-3 on MUC1 Expression

MCF-7 cells were treated with NM-3 (2-(8-hydroxy-6-methoxy-1-oxo-1 H-2-benzopyran-3-yl) propionic acid) at 100-400 µg/ml for 48 hr. DF3 antigen levels were visualized by immunoblot analysis with DF3 MAb (Kufe, U.S. Patent 5,506,343 ).

A decrease in the intracellular levels of DF3 antigen of NM-3 treated cells relative to non-treated cells was observed. Similar results were found in ZR-75-1 and BT-20 cell lines. The NM-3 mediated decrease of intracellular DF3 antigen was shown to be both dose- and time dependent. To determine whether NM-3 impaired the extracellular localization of the MUC1 DF3 antigen, the antigen levels in cell culture medium supernatants and on MCF-7 cells after NM-3 treatment were investigated. The levels of DF3 antigen were reduced in both localizations. These findings suggest that NM-3 inhibits MUC1 protein expression.

Hybridization studies were performed to determine whether the effect of NM-3 on MUCI-expression was detectable at the transcriptional level. A ³²P-labaelled DF3 DNA probe hybridized to two transcripts of 4.5 and 7.0 kb in total cellular RNA after NM-3 treatment on MCF-7 cells for 48 hr. The levels of both mRNA were decreased relative to controls wherein MCF-7 cells were incubated in the absence of NM-3. The same results were observed using ZR-75-1 and BT-20 cell lines. These findings suggest that DF3 expression is regulated at the transcriptional level after NM-3 treatment on those cell lines.

To determine whether NM-3 inhibits the expression of cellular surface proteins as well, the level of epidermal growth factor receptor (EGF-R) expression after NM-3 treatment was tested on MCF-7, ZR-75-1 and BT-20 cell lines. Compared to controls wherein cells were incubated in the absence of NM-3, there were no detectable changes in EGF-R expression after NM-3 treatment. These results indicate a selective effect of NM-3 on MUC1 expression without inhibition of surface molecular expression.

### REFERENCES

Barry & Sharkey, Hum. Pathol., 16:225-7,1985.
Brunner et al., Nature, 385:829-833, 1997.
Daniels & Reynolds, Mol. Cell. Biol., 19:3614-23,1999.
Dawson et al., Science 266:776, 1994.
Duffy, in Vaccine Preparation Techniques, Noyes Data Corporation of Park Ridge, N.J., 1980.
Finn et al., Immunol. Rev. 145:61-89, 1995.
Gansow, Int. J. Rad Appl. Instrum. [B], Nucl. Med. Biol. 18:369-381, 1991.
Goding, In: Monoclonal Antibodies: Principles and Practice, 2d. ed., Orlando, Fla., Academic Press, pp. 60-61, 1986.
Itzkowitz et al., Cancer, 66:1960-6, 1990.
Jawhari et al., J. Pathol., 189:180-85, 1999.
Jones et al., Nature 321:522-525, 1986.
Kufe et al., Hybridoma, 3:223-232, 984.
Li et al., Mol Cell Biol., 18:7216-24, 1998.
Li et al., J. Biol. Chem., 276:6061-64, 2001.
Li et al., J. Biol Chem, e-publication manuscript C100359200, August 1, 2001(a).
Li & Kufe, Biochem Biophys. Res. Commun., 281,440-43,2001.
Ligtenburger et al., Cancer Res., 15:23 18-2324, 1992.
Molenaar et al., Cell, 86:391-399, 1996.
Novak & Dedhar, Cell Mol. Life Sci., 523-37, 1999.
Olsnes & Pihl, Pharmac. Ther. 25:355-381, 1981.
Pandey et al., Cancer Res., 55:4000-4003, 1995.
Pastan et al., Ann. Rev. Biochem. 61:331-354, 1992.
Perey et al., Cancer Res., 52:2563-68, 1992.
Pluckthun in The Pharmacology of Monoclonal Antibodies, vol. 113:269-315, Rosenburg and Moore eds. Springer-Verlag, New York, 1994.
Presta, Curr. Op. Struct Biol. 2:593-596, 1992.
Reddish et al., Cancer Immunol. Immunother., 42:303-9, 1996.
Reynolds et al., Mol. Cell. Biol. 9:629-38, 1989.
Reynolds et al., Mol. Cell. Biol., 14:8333-41, 1994.
Riechmann et al., Nature 332:323-329, 1988.
Ring et al., Human Genet., 104:326-32, 1999.
Sambrook et al., Molecular Cloning: A Laboratory Manual, Cold Spring Harbor Laboratory Press, Cold Spring Harbor, N.Y. 1989.
Shimazui et al., Cancer Res., 56:4154-58, 1996.
Smith & Waterman, Adv. Appl. Math., 2:482-489, 1981.
Strouss & Decker, Crit. Rev. Biochem. Mol. Biol., 27:57-92, 1992.
Takeichi, Annu. Rev. Biochem., 59:237-52,1990.
Thorpe et al., Immunological Rev. 62:119-158, 1982.
Thorpe et al., Monoclonal Antibodies in Clinical Medicine, pp. 168-190 Academic Press, NY 1982(a).
Waldmann, Science, 252:1657,1991.
Yamamoto et. al., J. Biol. Chem. 272:12492-94, 1997.
Yuan et al., PNAS, 94:1437-1440, 1997.
Zrihan-Licht, et al., FEBS Let., 356:130-36, 1994.
U.S. Pat. No. 4,675,382
U.S. Pat. No. 4,894,443,
U.S. Pat. No. 5,506,343

## Claims

1. Use of a MUC1 extracellular domain antagonist for the manufacture of a medicament to inhibit the proliferation of MUC1-expressing cancer cells, wherein said MUC1 extracellular domain antagonist is:
i) the polypeptide of SEQ ID NO: 1, SEQ ID NO: 4 or SEQ ID NO: 5; or
ii) an antibody or fragment thereof that binds to an epitope within the amino acid sequence of SEQ ID NO: 1.

2. The use of the MUC1 extracellular domain antagonist of claim 1 wherein said medicament is formulated to be administered in combination with a chemotherapeutic agent or radiation.

3. The use of the MUC1 extracellular domain antagonist of claim 1, wherein said antibody is a monoclonal antibody, in particular wherein said monoclonal antibody is a humanized or a human antibody; or wherein said antibody is a bispecific antibody.

4. The use of the MUC1 extracellular domain antagonist of claim 1, wherein said antibody or fragment thereof is conjugated to a chemotherapeutic agent, to a radioisotope, to a toxin, to a cytokine, to an antimetabolite, to an anthracycline, to a vinca alkaloid, to an antibiotic, to an alkylating agent, or to an Fc region of an IgG1 immunoglobulin.

5. The use of the MUC1 extracellular domain antagonist of claim 1, wherein said medicament is administered as a composition comprising said antagonist and a pharmaceutically acceptable carrier.

6. The use of the MUC1 extracellular domain antagonist according to claim 2, wherein said chemotherapeutic agent is selected from alkylating agents, intercalating topoisomerase II inhibitors, non-intercalating topoisomerase II inhibitors, antimetabolites, tubulin interactive agents, anti-hormonal agents, ornithine decarboxylase inhibitors and tyrosine kinase inhibitors.

7. Polypeptide of SEQ ID NO: 1, SEQ ID NO: 4 or SEQ ID NO: 5 and a pharmaceutically acceptable carrier for use as pharmaceutical, wherein said polypeptide is a MUC1 extracellular domain antagonist.

8. A method of identifying a compound that inhibits the binding of ligands to the extracellular domain of MUC1, the method comprising the steps of:
(a) providing a polypeptide comprising the sequence of SEQ ID. NO: I or SEQ ID NO: 5;
(b) contacting a test compound and an antibody that binds to an epitope within SEQ ID NO: 4; and
(c) determining whether the binding of said antibody to the polypeptide is decreased relative to an appropriate control.

9. A method of identifying a compound that inhibits the proliferation of MUC1-expressing cancer cells, the method comprising the steps of:
(a) contacting a population of MUC1-expressing cancer cells obtained previously with a test compound and an antibody that binds to an epitope within SEQ ID NO: 5;
(b) determining whether the proliferation of said population of MUC1-expressing cancer cells is decreased by comparison to an appropriate control.

10. A method of identifying a compound that downregulates the expression the extracellular domain of MUC1, the method comprising the steps of:
(a) contacting a population of MUC1-expressing cancer cells obtained previously with a test compound;
(b) utilizing an antibody directed against the extracellular domain of MUC1 to identify polypeptides comprising the extracellular domain of MUC1 in the extracellular domain of MUC1-expressing cancer cells; and
(c) determining whether the expression of polypeptides comprising the extracellular domain of MUC1 is decreased in comparison to controls wherein the test compound was excluded.

11. The use of claim 1, wherein the cancer cells are from a tumor of the brain, lung, liver, spleen, kidney, lymph node, small intestine, pancreas, blood cells, colon, stomach, breast, endometrium, prostate, testicle, ovary, skin, head and neck, esophagus or bone marrow.

## Patentansprüche

1. Verwendung eines Antagonisten der extrazellulären Domäne von MUC 1 für die Herstellung eines Medikaments zur Inhibierung der Proliferation von MUC1 exprimierenden Krebszellen, wobei der Antagonist der extrazellulären Domäne von MUC1 ist:
i) das Polypeptid der SEQ ID NR: 1, SEQ ID NR: 4 oder SEQ ID NR: 5; oder
ii) ein Antikörper oder Fragment davon der/das an ein Epitop innerhalb der Aminosäuresequenz von SEQ ID NR: 1 bindet.

2. Verwendung des Antagonisten der extrazellulären Domäne von MUC1 nach Anspruch 1, wobei das Medikament formuliert ist für die Verabreichung in Kombination mit einem chemotherapeutischen Agens oder Bestrahlung.

3. Verwendung des Antagonisten der extrazellulären Domäne von MUC1 nach Anspruch 1, wobei der Antikörper ein monoklonaler Antikörper ist, insbesondere wobei der monoklonale Antikörper ein humanisierter oder humaner Antikörper ist; oder wobei der Antikörper ein bispezifischer Antikörper ist.

4. Verwendung des Antagonisten der extrazellulären Domäne von MUC 1 nach Anspruch 1, wobei der Antikörper oder ein Fragment davon konjugiert ist mit einem chemotherapeutischen Agens, mit einem Radioisotop, mit einem Toxin, mit einem Zytokin, mit einem Antimetabolit, mit einem Antrazyklin, mit einem Vincaalkaloid, mit einem Antibiotikum, mit einem alkylierenden Agens, oder mit einer Fc-Region eines IgG1-Immunglobulins.

5. Verwendung des Antagonisten der extrazellulären Domäne von MUC 1 nach Anspruch 1, wobei das Medikament als Zusammensetzung verabreicht wird, die den Antagonisten und einen pharmazeutisch verträglichen Träger umfasst.

6. Verwendung des Antagonisten der extrazellulären Domäne von MUC 1 nach Anspruch 2, wobei das chemotherapeutische Agens ausgewählt ist aus alkylierenden Agenzien, interkalierenden Topoisomerase-II-Inhibitoren, nicht-interkalierenden Topoisomerase-II-Inhibitoren, Antimetaboliten, Tubulin interaktiven Agenzien, antihormonellen Agenzien, Omithin-Dekarboxylase-Inhibitoren und Tyrosin-Kinase-Inhibitoren.

7. Polypeptid der SEQ ID NR: 1, SEQ ID NR: 4 oder SEQ ID NR: 5 und ein pharmazeutisch verträglicher Träger zur Verwendung als Pharmazeutikum, wobei das Polypeptid ein Antagonist der extrazellulären Domäne von MUC1 ist.

8. Verfahren zur Identifizierung einer Verbindung, die die Bindung von Liganden an die extrazelluläre Domäne von MUC1 inhibiert, wobei das Verfahren die Schritte umfasst:
(a) Bereitstellen eines Polypeptids, das die SEQ ID NR: 1 oder SEQ ID NR: 5 umfasst;
(b) In Kontakt bringen einer Testverbindung und eines Antikörpers, der an ein Epitop innerhalb von SEQ ID NR: 4 bindet; und
(c) Bestimmen, ob die Bindung des Antikörpers an das Polypeptid erniedrigt ist, relativ zu einer geeigneten Kontrolle.

9. Verfahren zur Identifizierung einer Verbindung, die die Proliferation von MUC1-expremierenden Krebszellen hemmt, wobei das Verfahren die Schritte umfasst:
(a) In Kontakt bringen einer Population von MUC 1 exprimierenden Krebszellen, die zuvor erhalten wurden, mit einer Testverbindung und einem Antikörper, der an ein Epitop innerhalb von SEQ ID NR: 5 bindet;
(b) Bestimmen, ob die Proliferation der Population von MUC 1-exprimierenden Krebszellen erniedrigt ist im Vergleich zu einer geeigneten Kontrolle.

10. Verfahren zur Identifizierung einer Verbindung, die die Expression der extrazellulären Domäne von MUC 1 herunterreguliert, wobei das Verfahren die Schritte umfasst:
(a) In Kontakt bringen einer Population von MUC 1 exprimierenden Krebszellen, die zuvor erhalten wurden, mit einer Testverbindung;
(b) Verwendung eines Antikörpers, der gegen die extrazelluläre Domäne von MUC1 gerichtet ist, zur Identifizierung von Polypeptiden, die die extrazelluläre Domäne von MUC1 in den Krebszellen umfassen, die die extrazelluläre Domäne von MUC1 exprimieren; und
(c) Bestimmen, ob die Expression von Polypeptiden, die die extrazelluläre Domäne von MUC1 umfassen, erniedrigt ist im Vergleich zu Kontrollen, wobei die Testverbindung ausgeschlossen wurde.

11. Verwendung nach Anspruch 1, wobei die Krebszellen von einem Tumor des Gehirns, der Lunge, der Leber, der Milz, der Niere, des Lymphknotens, des Dünndarms, der Bauchspeichedrüse, der Blutzellen, des Dickdarms, des Magens, der Brust, der Gebärmutterschleimhaut, der Prostata, der Hoden, der Eierstöcke, der Haut, des Kopfes und Halses, der Speiseröhre und des Knochenmarks sind.

## Revendications

1. Utilisation d'un antagoniste de domaine extracellulaire de MUC1 pour la fabrication d'un médicament destiné à inhiber la prolifération des cellules cancéreuses exprimant MUC1, dans laquelle ledit antagoniste de domaine extracellulaire de MUC1 est :
i) le polypeptide de SEQ ID N: 1, SEQ ID N: 4 ou SEQ ID N: 5 ; ou
ii) un anticorps ou un fragment de celui-ci qui se lie à un épitope à l'intérieur de la séquence d'acides aminés de SEQ ID N: 1.

2. Utilisation d'un antagoniste de domaine extracellulaire de MUC1 selon la revendication 1, dans laquelle ledit médicament est formulé pour être administré en combinaison avec un agent chimiothérapique ou les rayons.

3. Utilisation d'un antagoniste de domaine extracellulaire de MUC1 selon la revendication 1, dans laquelle ledit anticorps est un anticorps monoclonal, en particulier dans laquelle ledit anticorps monoclonal est un anticorps humanisé ou un anticorps humain ; ou dans laquelle ledit anticorps est un anticorps bispécifique.

4. Utilisation d'un antagoniste de domaine extracellulaire de MUC1 selon la revendication 1, dans laquelle ledit anticorps ou un fragment de celui-ci est conjugué à un agent chimiothérapique, à un radio-isotope, à une toxine, à une cytokine, à un antimétabolite, à une anthracycline, à un vinca-alcaloïde, à un antibiotique, à un agent alkylant, ou à une région Fc d'une immunoglobuline IgGI.

5. Utilisation d'un antagoniste de domaine extracellulaire de MUC1 selon la revendication 1, dans laquelle ledit médicament est administré sous la forme d'une composition comprenant ledit antagoniste et un véhicule pharmaceutiquement acceptable.

6. Utilisation d'un antagoniste de domaine extracellulaire de MUC1 selon la revendication 2, dans laquelle ledit agent chimiothérapique est choisi parmi les agents alkylants, les inhibiteurs de topo-isomérase II intercalants, les inhibiteurs de topo-isomérase II non intercalants, les antimétabolites, les agents interactifs avec la tubuline, les agents anti-hormonaux, les inhibiteurs d'ornithine décarboxylase et les inhibiteurs de tyrosine kinase.

7. Polypeptide de SEQ ID N: 1, SEQ ID N: 4 ou SEQ ID N: 5 et véhicule pharmaceutiquement acceptable pour l'utilisation en tant que produit pharmaceutique, dans lequel ledit polypeptide est un antagoniste de domaine extracellulaire de MUC1.

8. Procédé d'identification d'un composé qui inhibe la liaison des ligands au domaine extracellulaire de MUC1, le procédé comprenant les étapes consistant à :
(a) fournir un polypeptide comprenant la séquence de SEQ ID N: 1 ou SEQ ID N: 5;
(b) mettre en contact un composé test et un anticorps qui se lie à un épitope à l'intérieur de SEQ ID N: 4 ; et
(c) déterminer si la liaison dudit anticorps au polypeptide est réduite par rapport à un témoin approprié.

9. Procédé d'identification d'un composé qui inhibe la prolifération des cellules cancéreuses exprimant MUC1, le procédé comprenant les étapes consistant à :
(a) mettre en contact une population de cellules cancéreuses exprimant MUC1, obtenues au préalable avec un composé test et un anticorps qui se lie à un épitope à l'intérieur de SEQ ID N: 5 ;
(b) déterminer si la prolifération de ladite population des cellules cancéreuses exprimant MUC1 est réduite par rapport à un témoin approprié.

10. Procédé d'identification d'un composé qui régule à la baisse l'expression du domaine extracellulaire de MUC1, le procédé comprenant les étapes consistant à :
(a) mettre en contact une population de cellules cancéreuses exprimant MUC1, obtenues au préalable avec un composé test ;
(b) utiliser un anticorps dirigé contre le domaine extracellulaire de MUC1 pour identifier les polypeptides comprenant le domaine extracellulaire de MUC1 dans les cellules cancéreuses exprimant le domaine extracellulaire de MUC1; et
(c) déterminer si l'expression des polypeptides comprenant le domaine extracellulaire de MUC1 est réduite par rapport aux témoins dans lesquels le composé test a été exclu.

11. Utilisation selon la revendication 1, dans laquelle les cellules cancéreuses proviennent d'une tumeur du cerveau, du poumon, du foie, de la rate, du rein, du noeud lymphatique, de l'intestin grêle, du pancréas, des cellules sanguines, du colon, de l'estomac, du sein, de l'endomètre, de la prostate, des testicules, des ovaires, de la peau, de la tête et du cou, de l'oesophage ou de la moelle osseuse.
